(19) Europäisches Patentamt
European Patent Office
Office européen des brevets

(11) Veröffentlichungsnummer: **0 275 888 B1**

(12) # EUROPÄISCHE PATENTSCHRIFT

(45) Veröffentlichungstag der Patentschrift: **06.04.94**

(21) Anmeldenummer: **88100223.2**

(22) Anmeldetag: **11.01.88**

Die Akte enthält technische Angaben, die nach dem Eingang der Anmeldung eingereicht wurden und die nicht in dieser Patentschrift enthalten sind.

(51) Int. Cl.[5]: **C07D 471/04**, C07D 487/04, C07D 471/10, C07D 487/10, A61K 31/435, A61K 31/495, //(C07D471/04,235:00,221:00), (C07D487/04,239:00,335:00), (C07D487/04,241:00,235:00)

(54) **Neue tricyclische Benzimidazole, Verfahren zu ihrer Herstellung und Verwendung als Arzneimittel.**

(30) Priorität: **17.01.87 DE 3701277**

(43) Veröffentlichungstag der Anmeldung: **27.07.88 Patentblatt 88/30**

(45) Bekanntmachung des Hinweises auf die Patenterteilung: **06.04.94 Patentblatt 94/14**

(84) Benannte Vertragsstaaten: **AT BE CH DE ES FR GB GR IT LI LU NL SE**

(56) Entgegenhaltungen:
**EP-A- 0 001 279** **EP-A- 0 186 010**
**EP-A- 0 189 103** **EP-A- 0 207 483**
**DE-A- 3 410 168** **GB-A- 2 082 580**

**JOURNAL OF MEDICINAL CHEMISTRY, Band 289, 1986, Seiten 972-978, American Chemical Society, Washington, US; S.W.SCHNELLER et al.**

(73) Patentinhaber: **BOEHRINGER MANNHEIM GMBH**

**D-68298 Mannheim(DE)**

(72) Erfinder: **Mertens, Alfred, Dr. rer. nat.**
**Beethovenstrasse 20**
**D-6905 Schriesheim(DE)**
Erfinder: **von der Saal, Wolfgang, Dr. rer. nat.**
**Neuer Burgweg 3**
**D-6940 Weinheim(DE)**
Erfinder: **Böhm, Erwin, Dr. med.**
**Hinterer Rindweg 37**
**D-6802 Ladenburg(DE)**
Erfinder: **Strein, Klaus, Prof.**
**Eichenstrasse 45**
**D-6944 Hemsbach(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

MONATSHEFTE FUER CHEMIE, Band 117, 1986, Seiten 879-882, Springer-Verlag, Wien, AT, G.R. RODGERS et al.

CHEMICAL ABSTRACTS, Band 70, 1969, Seite 315, Zusammenfassung 28870c, Columbus, Ohio, US; CH.SH.KADYROV et al.,

CHEMICAL ABSTRACTS, Band 79, 1973, Seite 400, Zusammenfassung 126396h, Columbus Ohio, US; I.G.IL'INA et al.

ARCHIV DER PHARMAZIE, Band 308, Nr. 12, 1975, Seiten 977-980, Weinheim, DE; E. LEBENSTEDT et al.,

JOURNAL OF CHEMICAL RESEARCH, (S), 1986, Seiten 158-159, London, GB; S.RAJAPPA et al.,

CHEMICAL ABSTRACTS, Band 106, 1987, Seite 649, Zusammenfassung 102183m, Columbus, Ohio, US; D.R.SHRIDHAR et al.,

CHEMICAL ABSTRACTS, Band 97, 1982, Seite 572, Zusammenfassung 38906r, Columbus, Ohio, US; S.KUMAR et al.: "Possible anthelmintic agents: syntheses of various imidazoquinazolinone carbamates"

JOURNAL OF HETEROCYCLIC CHEMISTRY, Band 21, Mai-Juni 1984, Seiten 791-795, Tampa, US; S.W.SCHNELLER et al.: "The synthesis of lin-benzoreumycin, linbenzo-1-methylxanthine, and linbenzo-1,9-dimethylxanthine"

## Beschreibung

Die vorliegende Erfindung betrifft neue tricyclische Benzimidazole der allgemeinen Formel I,

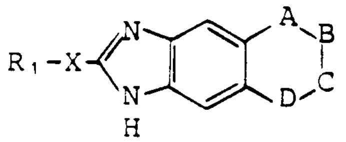

(I),

in welcher

$R_1$ einen Phenylring der allgemeinen Formel II darstellt,

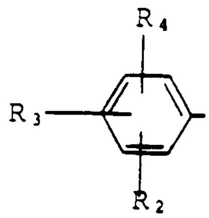

(II),

wobei $R_2$, $R_3$, $R_4$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-Alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe durch ein Schwefel- oder Sauerstoffatom ersetzt sein kann, eine Alkylcarbonylamino, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Hydroxyalkyl-, Dialkylamino-, 1-Imidazolyl-, Trifluormethyl-, Cyan-, Hydroxysulfonyl-, Bishydroxyalkylamino-, 1-Pyrrolidino-, 1-Piperidino-, 4-Morpholino-, 4-Thiomorpholino-, 1-Pyrazolyl-, 1-Triazolyl-, 2-Oxopyrrolidin-yl-, 2-Oxopiperidin- oder Pyrrolylgruppe sein können, und die zuvor genannten Alkylteile der bei $R_2$, $R_3$ und $R_4$ genannten Substituenten 1 - 5 Kohlenstoffatome enthalten,

oder $R_1$ einen Naphthyl- oder Methylendioxyphenylrest,

oder einen gesättigten oder ungesättigten heterocyclischen Fünfring mit 1 - 4 Heteroatomen oder einen heterocyclischen Sechsring mit 1 - 5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere $C_1$-$C_6$-Alkyl-, $C_1$-$C_6$-Alkoxy-, $C_1$-$C_6$-Alkylmercapto-, Hydroxy-, Oxo-, Nitro-, Amino-, Halogen- oder Cyangruppen substituiert oder mit einem Phenylring zu einem Bicyclus kondensiert sein können,

oder für den Fall, daß X eine Bindung darstellt, $R_1$ neben den oben genannten Gruppen auch ein Wasserstoffatom, eine $C_1$-$C_{10}$-Alkyl-, $C_3$-$C_7$-Cycloalkyl-, $C_2$-$C_{10}$-Alkenyl-, $C_3$-$C_7$-Cycloalkenyl-, $C_2$-$C_{10}$-Alkinyl-, Halogenalkyl-, Alkoxyalkyl-, Carboxyalkyl-, Alkoxycarbonylalkyl-, Aminoalkyl-, Hydroxyalkyl-, eine Hydroxy-, Mercapto-, Amino-, Alkylmercapto-, Alkylcarbonylamino-, Formylamino-, Alkylsufonylamino-, Formylaminoalkyl-, Alkoxycarbonylaminoalkyl-, Alkylsulfonylaminoalkyl-, Trifluormethyl- oder eine Alkylcarbonylaminoalkylgruppe bedeutet, wobei die zuvor unter $R_1$ genannten Alkyl- oder Alkoxygruppen 1-6 Kohlenstoffatome enthalten,

X eine Bindung, eine $C_1$-$C_4$-Alkylen-, die Vinylen-, die Iminogruppe -NH- oder die Amidgruppe -CONH- bedeutet, und

A, B, C und D unabhängig voneinander Kohlenstoff- oder Stickstoffatome bedeuten, wobei ein Atom von A, B, C oder D ein Stickstoffatom bedeutet und die anderen drei Atome Kohlenstoffatome darstellen, und A, B, C oder D durch Wasserstoff, $C_1$-$C_6$-Alkyl- oder $C_3$-$C_7$-Cycloalkylgruppen substituiert sein können, wobei ein Kohlenstoffatom durch eine Hydroxy- oder Oxogruppen substituiert ist oder Bestandteil eines 3- bis 7-gliedrigen Spirocyclus sein kann, und der A, B, C und D enthaltende Sechsring gesättigt, teilweise ungesättigt und ungesättigt sein kann,

deren Tautomere und deren physiologisch vertraegliche Salze anorganischer oder organischer Saeuren und Verfahren zu ihrer Herstellung, sowie diese Verbindungen enthaltende Arzneimittel.

Fuer den Fall, daß die Verbindungen der allgemeinen Formel I ein asymmetrisches Kohlenstoffatom enthalten, sind auch die optisch aktiven Verbindungen und racemischen Gemische Gegenstand der Erfindung.

Verbindungen mit ähnlicher Struktur sind aus dem Stand der Technik bekannt. In DE 3 410 168 werden Imidazo[4,5-h]isochinolin-4,6-dion-Derivate mit einer lang andauernden cardiotonen Wirkung beschrieben, die sich besonders zur Behandlung der Herzinsuffizienz eignen. In EP 0 186 010 werden Pyrrolobenzimidazole beschrieben, die in 2-Stellung des tricyclischen Ringsystems durch eine Phenylgruppe substituiert sind. Diese Verbindungen zeichnen sich insbesondere aus durch ihre herzkraftsteigernde und blutdrucksenkende Wirkung. In EP 0 189 103 sind die entsprechenden heterocyclisch substituierten Pyrrolobenzimidazole beschrieben, die ähnliche pharmakologische Wirkungen wie die in EP 0 186 010 beschriebenen Phenylsubstituierten Derivate aufweisen. Weiterhin sind aus EP 0 207 483 Derivate mit vergleichbarer Wirkung, wie in EP 0 186 010 und EP 0 189 103 beschrieben, bekannt.

Die neuen Verbindungen der allgemeinen Formel I weisen wertvolle pharmakologische Eigenschaften auf, insbesondere hemmen sie die Aggregation der Thrombozyten und Erythrozyten und/oder wirken blutdrucksenkend und/oder koennen die Herzkraft steigern.

Bedeutet $R_1$ einen Phenylring der allgemeinen Formel II so kann der Alkylteil der bei $R_2$, $R_3$ und $R_4$ genannten Substituenten 1-5 Kohlenstoffatome enthalten, vorzugsweise 1-4 Kohlenstoffatome. Diese Alkylteile können gerade oder verzweigt sein. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-, N-Methyl-trifluormethansulfonylamino-, N-Ethyl-trifluormethansulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylaminocarbonylamino-,Ethylaminocarbonylamino oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Hydroxymethyl-, Methylmercapto-, Ethylmercapto-, Methylsulfinyl-, Ethylsulfinyl-, Methylsulfonyl- oder Ethylsulfonylgruppe.

Bei Sulfonylgruppen, die durch cyclische Iminogruppen substituiert sein koennen, sind bevorzugt die Morpholino-, Pyrrolidino-, Piperidino- und Hexamethyleniminosulfonylgruppen.

Insbesondere sind bevorzugt fuer

$R_2$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethyloxy-oder Methoxycarbonylmethyloxygruppe, die Trifluormethylgruppe oder die 1-Imidazolylgruppe,

fuer $R_3$ Wasserstoff, eine Alkylgruppe mit 1-3 Kohlenstoffatomen, eine Alkoxy- oder Dialkylaminogruppe mit 1 oder 2 Kohlenstoffatomen in jedem Alkylteil oder ein Halogenatom und
fuer $R_4$ Wasserstoff oder die Methoxygruppe.

Der Phenylteil kann 1 bis 3 der genannten Substituenten tragen.

Bevorzugte monosubstituierte Phenylverbindungen sind die Hydroxy-, $C_1$-$C_3$ Alkyl-, $C_1$-$C_3$ Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, $C_1$-$C_3$ Dialkylamino-, $C_1$-$C_3$ Alkylmercapto-, $C_1$-$C_3$ Alkylsulfinyl-, $C_1$-$C_3$ Alkylsulfonyl-, $C_1$-$C_3$ Alkylsulfonyloxy- und die 1-Imidazolyl-phenyle,wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino-oder Morpholinogruppe substituierte Sulfonylgruppe, eine Alkylaminosulfonyl-, Alkylcarbonylamino-, Aminocarbonylamino-oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl-oder eine 1-Imidazolylgruppe, wobei die beiden Substituenten gleich oder verschieden sein koennen und in 2,3-, 2,4-, 2,5-, 2,6-, 3,4- und 3,5-Stellung bevorzugt jedoch in 2,4-, 2,5-und 3,4-Stellung stehen koennen und die vorgenannten Alkylreste, allein oder in Kombination mit anderen Resten, 1-3 C-Atome aufweisen koennen.

Bevorzugter trisubstituierter Phenylrest ist der 3,4,5-Trimethoxyphenylrest.

Bedeutet $R_1$ einen gesaettigten oder ungesaettigten heterocyclischen Fuenfring mit 1-4 Heteroatomen oder einen heterocyclischen Sechsring mit 1-5 Heteroatomen, wobei die Heteroatome der vorgenannten Fuenf- oder Sechsringe gleich oder verschieden sein koennen und Stickstoff, Sauerstoff oder Schwefel bedeuten und gegebenenfalls an einem oder mehreren Stickstoffatomen ein Sauerstoff tragen koennen, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,N'-Dioxy-pyrazin-, Pyrimidin-, N,N'-Dioxy-pyrimidin-, Pyridazin-, Oxazin-, Thiazin-, Triazin-, Tetrazin-, Pyridin-, N-Oxy-pyridin-, Piperidin-, Piperazin-, Morpholin- oder Thiomorpholinrest.

Alkyl-, Alkoxy- und Alkylmercapto-Substituenten in den heterocyclischen Fünf- und Sechsring können 1 - 6, vorzugsweise 1 - 4 Kohlenstoffatome enthalten, wobei diese Gruppen geradkettig oder verzweigt sein können. Bevorzugt ist der Methyl-, Ethyl-, Methoxy-, Ethoxy-, Methylmercapto- und Ethylmercaptorest.

Unter Halogen ist allgemein Fluor, Chlor und Brom, vorzugsweise Chlor zu verstehen.

Sind die gesaettigten oder ungesaettigten heterocyclischen Fuenf- und Sechsringe mit einem Phenylring kondensiert so sind bevorzugt der Indol-, Indazol-, Benzimidazol-, Chinolin-, Isochinolin-, Cinnolin-, Phthalazin-, Chinazolin-, Chinoxalin-, Benzofuran-, Benzothiophen-, Benzoxazol-, Benzisoxazol-, Benzothiazol- und der Benzisothiazolrest.

Bedeutet X eine Bindung und $R_1$ einen Alkyl-, Alkenyl- oder Alkinylrest, so sind darunter gerade oder verzweigte Ketten mit bis zu zehn C-Atomen zu verstehen.

Bevorzugt in diesem Sinne ist für $R_1$ der Methyl-, Ethyl-, Propyl-, Butyl-, Pentyl-, Hexyl-, Vinyl-, Propenyl- und Propionylrest. Bedeutet X eine Bindung und $R_1$ einen Cycloalkyl- oder Cycloalkenylrest, so sind darunter Ringe mit drei bis sieben Gliedern zu verstehen. Bevorzugt in diesem Sinne ist der Cyclopropyl-, Cyclobutyl-, Cyclopentyl-, Cyclohexyl-, Cyclopentenyl- und Cyclohexenylrest. Bedeutet X eine Bindung und $R_1$ einen Halogenalkyl-, Alkoxyalkyl-, Carboxyalkyl-, Alkoxycarbonylalkyl-, Aminoalkyl-, Hydroxyalkyl-, Alkoxycarbonylaminoalkyl-, Alkylsulfonylaminoalkyl-, Alkylmercapto-oder einen Alkylcarbonylaminorest, so koennen die Alkyl-oder Alkoxygruppen 1 bis 6 C-Atomen enthalten. Unter Halogen ist Fluor und Chlor, vorzugsweise Fluor zu verstehen.

Bevorzugt in diesem Sinne ist für $R_1$ der Trifluormethyl-, Ethoxy-, methyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Aminomethyl-, Aminoethyl-, Aminopropyl-, Aminobutyl-, Methylmercapto-, Ethylmercapto-, Propylmercapto-, Butylmercapto-, Acetylamino-, Propionylamino-, Butyloxycarbonylamino-, Methylsulfonylamino-, Formylaminopropyl-, Acetylaminopropyl-, Propionylaminopropyl- und der Methylsulfonylaminopropylrest.

Bedeutet in der allgemeinen Formel I X eine Alkylengruppe, so sind darunter Alkylengruppen mit 1-4 C-Atomen zu verstehen. Bevorzugt in diesem Sinne ist die Methylen- und Ethylengruppe.

Für die A, B, C und D enthaltenden heterocyclischen Sechsringe kommen sowohl die ungesättigten als auch die entsprechenden Dihydro- und Tetrahydro-Derivate in Frage.

Bevorzugte tricyclische Ringsysteme der Formel I sind 5,6,7,8-Tetrahydro-1H-imidazo[4,5-g]chinolin-6-on, 2,3,5,6,7,8-Hexahydro-1H-imidazo[4,5-g]chinolin-2,6-dion, 5,6,7,8-Tetrahydro-1H-imidazo[4,5-g]-isochinolin-5-on, 5,6-Dihydro-1H-imidazo[4,5-g]isochinolin-5-on, 5,6,7,8-Tetrahydro-1H-imidazo[4,5-g]-isochinolin-5,7-dion, 5,6,7,8-Tetrahydro-1H-imidazo-[4,5-g]chinolin-6-on, sowie deren im A, B, C und D enthaltenden Teilsystem durch Methyl- oder Hydroxygruppen substituierte Derivate.

Besonders bevorzugte Verbindungen sind Verbindungen der allgemeinen Formel I in der

$R_1$ den Phenylrest der allgemeinen Formel II bedeutet, in der

$R_2$ ein Wasserstoff, die Methansulfonyloxy-, Trifluormethansulfonyloxy-, Methansulfonylamino-, Trifluormethansulfonylamino-, Methansulfonylmethylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Methyl-, Methoxy-, Propargyloxy-, Cyanmethyloxy-, Methoxycarbonylmethyloxy-,Cyan-, Chlor-, Nitro-, Amino-, Dimethylamino-, Trifluormethyl- oder die 1-Imidazolylgruppe,

$R_3$ Wasserstoff, die Methyl-, Methoxy-, Dimethylamino- oder Chlorgruppe bedeutet,

$R_4$ Wasserstoff oder die Methoxygruppe ist

oder

$R_1$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxypyridin-, Pyrazin-, N,N'-Dioxy-pyrazin-, Pyrimidin-, N,N'-Dioxypyrimidin-, Pyridazin-, Oxazin-, Thiazin-, Triazin-, Tetrazin-, Piperidin-, Piperazin-, Morpholin- oder Thiomorpholinrest darstellt, sowie deren Methyl-, Ethyl-, Methoxy-` Ethoxy-, Methylmercapto-, Ethylmercapto- und chlorsubstituierten Derivate, oder den Naphthalin, Indol, Indazol-, Chinolin- oder Isochinolinrest bedeutet,

oder

$R_1$ fuer den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Amino-, Acetylamino-, Formylaminogruppe bedeutet,

X eine Bindung, eine Methylen-, Vinylen-, Imino- oder Amidgruppe-CONH- bedeutet und ein Atom von A, B, C oder D Stickstoff bedeutet und die anderen Kohlenstoffatome darstellen, die durch Wasserstoff oder Methylgruppen substituiert sein koennen und die Kohlenstoffatome zusaetzlich eine Hydroxy- oder Oxogruppen tragen oder Bestandteil eines Spirocyclus mit 5 C-Atomen sein koennen.

Bevorzugt kommen Verbindungen der Formel I in Frage, in der

$R_1$ eine Phenylgruppe, die gegebenenfalls durch Methoxy, Ethoxy, Chlor, Methyl, Hydroxy, Dimethylamino, Diethylamino, Imidazol-1-yl, Allyloxy, Cyan und Aminocarbonyl substituiert sein kann, eine Methylendioxyphenylgruppe oder eine Pyridyl-, Chinolinyl-, Pyridazinyl-, Pyrazolyl- oder Thienylgruppe darstellt, sowie deren durch Methyl substituierten Derivate, oder

$R_1$ für den Fall, daß X eine Bindung darstellt, neben den genannten Gruppen auch eine Methyl-, n-Hexyl- und Hydroxygruppe bedeutet.

Die Verbindungen der allgemeinen Formel I koennen nach an sich in der Literatur bekannten Verfahren hergestellt werden (G.W.H. Cheeseman, R.F. Cookson in The Chemistry of Heterocyclic Compounds, Ed.: A. Weissberger, E. Taylor, Vol. 35, Seite 78-111, 1979 und P.N. Preston, D.M. Smith, G. Tennant, ebenda, Vol. 40, Part 1, Seite 1-286, 1981).

Besonders vorteilhaft sind die in den folgenden Schemeta gezeigten Verfahren, bei denen man

a) Verbindungen der allgemeinen Formel III,

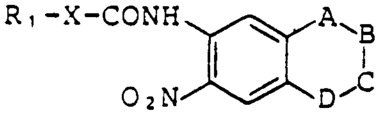

(III),

oder der allgemeinen Formel IV,

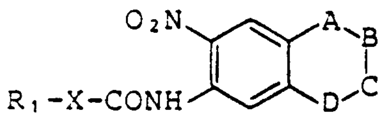

(IV),

in denen $R_1$, X, A, B, C und D die angegebene Bedeutung haben, hydriert und zum Benzimidazol cyclisiert, oder

b) Verbindungen der allgemeinen Formel V,

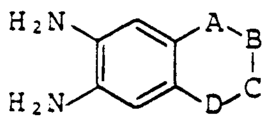

(V),

in denen A, B, C und D die angegebene Bedeutung haben, mit Verbindungen der allgemeinen Formel VI,

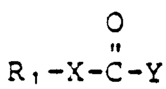

(VI),

in denen $R_1$ und X die angegebene Bedeutung haben und Y ein leicht abspaltbarer Rest ist, umsetzt, oder

c) fuer den Fall, daß $R_1$ in Verbindungen der allgemeinen Formel I eine Amino-, Hydroxy- oder Mercaptogruppe und X ein Valenzstrich bedeutet, Verbindungen der allgemeinen Formel V, in denen A, B, C und D die angegebene Bedeutung haben, mit Reagenzien umsetzt, die Carbonyl-, Thiocarbonyl- oder Iminogruppen uebertragen, wie z.B. Phosgen, Thiophosgen, 1,1'-Carbonyldiimidazol, Chlorameisensaeureester, Harnstoff oder Bromcyan.

Die in Verfahren a genannte Reduktion wird vorzugsweise in einem Loesungsmittel oder Loesungsmittelgemisch wie Wasser, Methanol, Ethanol, Eisessig, Essigsaeureethylester oder Dimethylformamid mit Wasserstoff in Gegenwart eines Katalysators wie Raney-Nickel, Platin oder Palladium/Kohle, mit Metallen wie Eisen, Zinn oder Zink in Gegenwart einer Saeure, mit Salzen wie Eisen(II)sulfat, Zinn(II)chlorid, Natriumsulfid, Natriumhydrogensulfit oder Natriumdithionit oder mit Hydrazin in Gegenwart von Raney-Nickel bei Temperaturen zwischen 0 und 100 °C, vorzugsweise jedoch bei Raumtemperatur, durchgefuehrt. Dabei entstehen meist unmittelbar die cyclisierten Verbindungen der allgemeinen Formel I .

Die Cyclisierung kann gewuenschtenfalls vervollstaendigt werden, indem man nach der Reduktion vorzugsweise in einem Loesungsmittel oder Loesungsmittelgemisch wie Ethanol, Isopropanol, Eisessig, Benzol, Toluol, Chlorbenzol, Glycol, Ethylenglycoldimethylether, Sulfolan oder Dimethylformamid auf Temperaturen zwischen50 und 220 °C, vorzugsweise jedoch auf die Siedetemperatur des Reaktionsgemisches, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxid, Natriumethylat oder Kalium-tert.-butylat erhitzt . Die Cyclisierung kann jedoch auch ohne Loesungsmittel und/oder Kondensationsmittel durchgefuehrt werden.

Unter den in Verfahren b genannten Verbindungen der allgemeinen Formel VI versteht man Aldehyde, Carbonsaeuren, Saeurehalogenide wie Saeurechloride, Carbonsaeureester wie Methyl- und Ethylester, Carbonsaeureamide und andere aktivierte Carbonsaeurederivate sowie Anhydride.

Ist die Verbindung der allgemeinen Formel VI ein Aldehyd, so findet die Umsetzung mit Verbindungen der allgemeinen Formel V unter oxidierenden Bedingungen statt, vorzugsweise in alkoholischem Medium unter Erwaermen zum Rueckfluß in Gegenwart von Luftsauerstoff und katalytischen Mengen Saeure, wie Toluolsulfonsaeure, oder in Gegenwart von Luftsauerstoff und eines Katalysators wie Braunstein in saurem Milieu wie z.B. in Eisessig bei Raumtemperatur.

Ist die Verbindung der allgemeinen Formel VI eine Carbonsaeure, so findet die Umsetzung mit Verbindungen der allgemeinen Formel V in Gegenwart eines wasserentziehenden Mittels, vorzugsweise in

Polyphosphorsaeure bei Temperaturen zwischen 50 und 250 °C, vorzugsweise zwischen 100 und 200 °C statt.

Ist die Verbindung der allgemeinen Formel VI ein Carbonsaeurederivat, so findet die Umsetzung mit Verbindungen der allgemeinen Formel V in einem inerten Loesungsmittel, vorzugsweise in Methylenchlorid oder Pyridin statt. Zur Vervollstaendigung der Cyclisierung erhitzt man anschließend in einem Loesungsmittel oder Loesungsmittelgemisch wie Ethanol, Isopropanol, Eisessig, Benzol, Chlorbenzol, Glycol, Diethylenglycoldimethylether, Sulfolan oder Dimethylformamid auf Temperaturen zwischen 50 und 250 °C, vorzugsweise jedoch auf die Siedetemperatur des Loesungsmittels oder Loesungsmittelgemisches, gegebenenfalls in Gegenwart einen Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxid, Kaliummethylat oder Kalium-tert.-butylat. Die Cyclisierung kann jedoch auch ohne Loesungsmittel und/oder Kondensationsmittel durchgefuehrt werden.

Die in Verfahren c beschriebenen Cyclisierungen der Verbindungen der allgemeinen Formel V zu Verbindungen der allgemeinen Formel I fuehrt man vorzugsweise so aus, daß man in eine salzsaure Loesung der Verbindungen der allgemeinen Formel V Phosgen einleitet oder Thiophosgen zugibt und bei Raumtemperatur stehen laeßt, oder die Verbindungen der allgemeinen Formel V mit Brom-cyan oder Harnstoff ohne Loesungsmittel erhitzt, oder die Verbindungen der allgemeinen Formel V mit 1,1′-Carbonyldiimidazol in einem inerten Loesungsmittel wie Dioxan zum Sieden erhitzt.

Die nach den Verfahren a - c erhaltene Verbindung der allgemeinen Formel I oder deren Tautomer kann anschließend gewuenschtenfalls in eine andere Verbindung der allgemeinen Formel I umgewandelt und/oder in ein physiologisch vertraegliches Salz einer organischen oder anorganischen Saeure ueberfuehrt werden.

Die Umwandlung von Verbindungen der allgemeinen Formel I zu anderen Verbindungen der allgemeinen Formel I trifft zum Beispiel zu

a) fuer die Umsetzung einer Verbindung der allgemeinen Formel I, in der $R_1$ eine Amino-, Aminoalkyl- oder cyclische Iminogruppe darstellt, oder einen mit einer Aminogruppe substituierten heterocyclischen Fuenf- oder Sechsring darstellt, oder einen Phenylring der allgemeinen Formel II darstellt, in der ein oder mehrere der Substituenten $R_2$, $R_3$, $R_4$ eine Aminogruppe darstellen, mit Carbonsaeuren oder mit aktivierten Carbonsaeurederivaten wie Anhydriden oder Saeurehalogeniden zu Formylamino- oder Alkylcarbonylaminoderivaten. Umsetzungen mit Carbonsaeuren fuehrt man vorzugsweise in Gegenwart eines wasserentziehenden Mittels wie z.B. Polyphosphorsaeure oder eines mit Wasser ein azeotropes Gemisch bildenden Loesungsmittels wie Benzol oder Toluol durch. Umsetzungen mit aktivierten Carbonsaeurederivaten werden vorzugsweise in inerten Loesungsmitteln wie Methylenchlorid oder Pyridin bei Temperaturen zwischen 0 °C und 250 °C, vorzugsweise jedoch bei der Siedetemperatur des Loesungsmittels durchgefuehrt;

b) fuer die Umsetzung von Verbindungen der allgemeinen Formel I, in der $R_1$ eine Amino-, Aminoalkyl- oder cyclische Iminogruppe darstellt, oder R einen mit einer Aminogruppe substituierten heterocyclischen Fuenf- oder Sechsring darstellt, wie er eingangs definiert ist, oder $R_1$ einen Phenylring der allgemeinen Formel II darstellt, in welcher einer der Substituenten $R_2$, $R_3$, $R_4$ eine Amino-, N-Alkylamino- oder Hydroxygruppe darstellt, mit einer Sulfonsaeure der allgemeinen Formel IX,

$R_7$-$SO_2OH$ (IX)

in der $R_7$ eine Alkylgruppe mit 1-3 Kohlenstoffatomen oder eine Trifluormethylgruppe darstellt oder mit einem reaktionsfaehigen Derivat hiervon, zu Verbindungen der allgemeinen Formel I, in welcher die besagten Amino-, Aminoalkyl-, cyclischen Imino-, N-Alkylamino- oder Hydroxygruppen sulfoniert sind.

Die Umsetzung wird zweckmaeßigerweise in einem Loesungsmittel oder Loesungsmittelgemisch wie Methylenchlorid, Ether, Tetrahydrofuran, Dioxan oder Benzol gegebenenfalls in Gegenwart eines saeurebindenden Mittels wie Natriumcarbonat, Triethylamin oder Pyridin, wobei die beiden letzteren gleichzeitig auch als Loesungsmittel verwendet werden koennen, in Gegenwart eines die Saeure aktivierenden oder wasserentziehenden Mittels wie Thionylchlorid oder Phosphorpentachlorid, vorzugsweise jedoch mit einem reaktionsfaehigen Derivat einer Verbindung der allgemeinen Formel IX, z.B. mit deren Anhydrid oder Halogenid wie Methansulfonsaeurechlorid oder Ethansulfonsaeurechlorid, vorzugsweise bei Temperaturen zwischen 0 und 100 °C, z.B. bei Temperaturen zwischen Raumtemperatur und 50 °C, durchgefuehrt;

c) fuer die Umwandlung von Verbindungen der allgemeinen Formel I, in der $R_1$ eine Phenylgruppe der allgemeinen Formel II darstellt, wobei einer der Substituenten $R_2$, $R_3$, $R_4$ eine Alkylmercapto- oder Alkylsulfenylmethylgruppe mit 1-3 Kohlenstoffatomen im Alkylteil ist, zu Verbindungen der allgemeinen

Formel I, in der $R_1$ eine Phenylgruppe und einer der Substituenten $R_2$, $R_3$, $R_4$ eine Alkylsulfinyl-, Alkylsulfonyl-, Alkylsulfinylmethyl- oder Alkylsulfonylmethylgruppe darstellt.

Diese Oxidation wird vorzugsweise in einem Loesungsmittel oder Loesungsmittelgemisch, z.B. in Wasser, Wasser/Pyridin, Aceton, Eisessig, verduennter Schwefelsaeure oder Trifluoressigsaeure, je nach dem verwendeten Oxidationsmittel zweckmaeßigerweise bei Temperaturen zwischen -80 und 100 °C durchgefuehrt.

Zur Herstellung einer Alkylsulfinyl- oder Alkylsulfinylmethylverbindung der allgemeinen Formel I wird die Oxidation zweckmaeßigerweise mit einem Aequivalent des verwendeten Oxidationsmittel durchgefuehrt, z.B. mit Wasserstoffperoxid in Eisessig, Trifluoressigsaeure oder Ameisensaeure bei 0 bis 20 °C oder in Aceton bei 0 bis 60 °C, mit einer Persaeure wie Perameisensaeure in Eisessig oder Trifluoressigsaeure bei 0 bis 50 °C oder mit m-Chlorperbenzoesaeure in Methylenchlorid oder Chloroform bei -20 °C bis 60 °C, mit Natriummetaperjodat in waessrigem Methanol oder Ethanol bei -15 bis 25 °C, mit Brom in Eisessig oder waessriger Essigsaeure, mit N-Brom-succinimid in Ethanol, mit tert.-Butylhypochlorit in Methanol bei -80 °C bis -30 °C, mit Jodbenzodichlorid in waessrigem Pyridin bei 0 bis 50 °C, mit Salpetersaeure in Eisessig bei 0 bis 20 °C, mit Chromsaeure in Eisessig oder in Aceton bei 0 bis 20 °C und mit Sulfurylchlorid in Methylenchlorid bei -70 °C, der hierbei erhaltene Thioether-Chlor-Komplex wird zweckmaeßigerweise mit waessrigem Ethanol hydrolysiert.

Zur Herstellung einer Alkylsulfonyl- oder Alkylsulfonylmethylverbindung der allgemeinen Formel I wird die Oxidation zweckmaeßigerweise mit einem bzw. mit zwei oder mehr Aequivalenten des verwendeten Oxidationsmittels durchgefuehrt, z.B. Wasserstoffperoxid in Eisessig, Trifluoressigsaeure oder in Ameisensaeure bei 20 bis 100 °C oder in Aceton bei 0 bis 60 °C, mit einer Persaeure wie Perameisensaeure, oder m-Chlorperbenzoesaeure in Eisessig, Trifluoressigsaeure, Methylenchlorid oder Chloroform bei Temperaturen zwischen 0 und 60 °C, mit Salpetersaeure in Eisessig bei 0 bis 20 °C, mit Chromsaeure oder Kaliumpermanganat in Eisessig, Wasser/Schwefelsaeure oder in Aceton bei 0 bis 20 °C:

d) fuer die Umwandlung von Verbindungen der allgemeinen Formel I, in der $R_1$ eine Phenylgruppe der allgemeinen Formel II darstellt, wobei einer der Substituenten $R_2$, $R_3$, $R_4$ eine Carboxyl- oder Hydroxysulfonylgruppe darstellt, zu Verbindungen der allgemeinen Formel I, in der einer der Substituenten $R_2$, $R_3$ und $R_4$ eine durch eine Amino-, Alkylamino- oder Dialkylaminogruppe substituierte Carbonyl- oder Sulfonylgruppe darstellt. Dies geschieht durch Umsetzung mit einem Amin $HNR_8R_9$ , wobei $R_8$ und $R_9$ gleich oder verschieden sein koennen und Wasserstoff oder $C_1$-$C_5$ Alkylgruppen bedeuten, oder mit einem reaktionsfaehigen Derivat hiervon. Vorteilhaft ist es, die Carboxylgruppe oder Hydroxysulfonylgruppe in ein reaktionsfaehiges Derivat, z.B. in einen Ester oder ein Saeurechlorid zu verwandeln und dann mit dem Amin $HNR_8R_9$ umzusetzen.

Die Umsetzung wird zweckmaeßigerweise in einem Loesungsmittel oder Loesungsmittelgemisch wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Saeure aktivierenden Mittels oder eines wasserentziehenden Mittels z.B. in Gegenwart von Chlorameisensaeureethylester, Thionylchlorid, Phosphortrichlorid, Phosphorpentoxid, N′N-Dicyclohexylcarbodiimid/N-Hydroxysuccinimid, N,N′-Carbonyldiimidazol oder N,N′-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen -25 und 250 °C, vorzugsweise jedoch bei Temperaturen zwischen -10 °C und der Siedetemperatur des verwendeten Loesungsmittels durchgefuehrt werden, desweiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzumg in einem entsprechenden Halogenid, z.B. dem Carbonsaeure- oder Sulfonsaeurechlorid, und einem entsprechenden Amin, wobei diesesgleichzeitig als Loesungsmittel dienen kann, und bei Temperaturen zwischen 0 und 50 °C durchgefuehrt,

e) fuer die Umwandlung von Verbindungen der allgemeinen Formel I, in der $R_1$ einen Phenylring der allgemeinen Formel II darstellt und einer der Substituenten $R_2$, $R_3$ oder $R_4$ die Cyangruppe darstellt zu Verbindungen der allgemeinen Formel I, in welcher $R_1$ einen Phenylring der allgemeinen Formel II darstellt und einer der Substituenten $R_2$, $R_3$ oder $R_4$ eine Alkoxycarbonylgruppe-, eine Aminocarbonylgruppe oder eine Carboxylgruppe bedeutet.

Diese Alkoholyse und/oder Hydrolyse wird entweder in Gegenwart einer Saeure wie Salzsaeure, Schwefelsaeure, Phosphorsaeure oder Trichloressigsaeure oder in Gegenwart einer Base wie Natriumhydroxid oder Kaliumhydroxid in einem geeigneten Loesungsmittel wie Wasser, Wasser/Methanol, Ethanol,Wasser/Ethanol, Wasser/Isopropanol oder Wasser/Dioxan bei Temperaturen zwischen -10 und

120 °C, z.B. bei Temperaturen zwischen Raumtemperatur und der Siedetemperatur des Reaktionsgemischens, durchgefuehrt;

f) fuer die Alkylierung von Verbindungen der allgemeinen Formel I, in welcher $R_1$ einen Phenylring der allgemeinen Formel II darstellt, in der einer der Substituenten $R_2$, $R_3$ oder $R_4$ eine Hydroxy- oder Mercaptogruppe bedeutet, oder in welcher $R_1$ einen mit einer Hydroxy- oder Mercaptogruppe substituierten heterocyclischen Ring darstellt oder in der X eine Bindung darstellt und $R_1$ eine Hydroxy- oder Mercaptogruppe bedeutet. Dabei entstehen die entsprechenden Alkylmercapto- oder Alkoxyverbindungen.

Die Reaktionen werden vorzugsweise in einem Loesungsmittel wie Aceton,Ether, Benzol, Toluol oder Dimethylformamid bei Temperaturen zwischen -30 °C und +100 °C, vorzugsweise bei Raumtemperatur in Gegenwart einer Base wie Kaliumcarbonat oder Natriumhydrid und eines Alkylierungsmittels wie Alkylhalogeniden oder Alkylsulfaten durchgefuehrt,

g) fuer die Reduktion von Verbindungen der allgemeinen Formel I, in welcher $R_1$ einen Pyridinring darstellt, zu Verbindungen der allgemeinen Formel I, in der $R_1$ den Piperidinring bedeutet. Diese Reduktionen fuehrt man vorzugsweise in alkoholischen Medium in Gegenwarte eines Katalysators wie Platin oder Palladium mittels Wasserstoff bei Normaldruck oder leicht erhoehtem Druck und einer Temperatur zwischen Raumtemperatur und 60 °C;

h) fuer die Hydrierung einer Vinylverbindung (X = -CH=CH-) in eine entsprechende Ethylverbindung (X = $-CH_2-CH_2-$). Die Hydrierung wird vorzugsweise in einem Loesungsmittel wie Wasser, Wasser/Ethanol, Methanol, Eisessig, Essigsaeureethylester oder Dimethylformamid zweckmaeßigerweise mit Wasserstoff in Gegenwart eines Hydrierungskatalysators wie Raney-Nickel, Platin oder Palladium/Kohle durchgefuehrt.

i) fuer die Oxidation eines Fuenf- oder Sechsringes mit einem oder mehreren Stickstoffatomen zu den entsprechenden N-Oxiden. Die Oxidation wird zweckmaeßig mit einem oder mehreren Aequivalenten des verwendeten Oxidationsmittels durchgefuehrt, z.B. mit Wasserstoffperoxid in Eisessig, Trifluoressigsaeure oder in Ameisensaeure bei 20 - 100 °C oder in Aceton bei 0 - 60 °C, mit einer Persaeure wie Perameisensaeure oder m-Chlorperbenzoesaeure in Eisessig, Trifluoressigsaeure, Methylenchlorid oder Choroform bei Temperaturen zwischen 0 und 60 °C;

j) fuer die Umwandlung von Verbindungen der allgemeinen Formel Ia

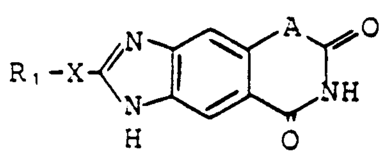

(Ia)

in der $R_1$ und X die angegebene Bedeutung haben und A ein Kohlenstoffatom mit zwei Alkylresten ist, zu Verbindungen der allgemeinen Formel Ib

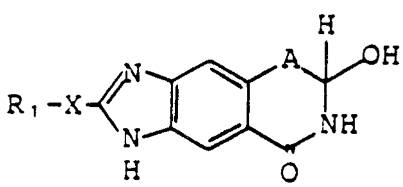

(Ib),

in der $R_1$ und X die angegebene Bedeutung haben und A ein Kohlenstoffatom mit zwei Alkylresten ist.

Die Umwandlung wird vorteilhaft in einem Loesungsmittel-oder Loesungsmittelgemisch wie Methanol, Ethanol, Dioxan, Tetrahydrofuran, Wasser oder Dimethylformamid in Gegenwart eines Reduktionsmittels wie Natriumborhydrid, Natriumcyanoborhydrid, Lithiumaluminiumhydrid oder anderen komplexen Hydriden oder mit Boran oder durch katalytische Hydrierung bei Temperaturen zwischen -20 °C und 100 °C durchgefuehrt.

k) fuer die Umwandlung von Verbindungen der allgemeinen Formel Ib zu Verbindungen der allgemeinen Formel Ic

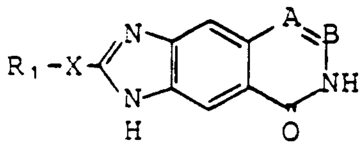

(Ic),

in der $R_1$ und X die angegebene Bedeutung haben und A und B Kohlenstoffatome darstellen, die durch eine Alkylgruppe substituiert sind.

Diese Umwandlung wird in einem Loesungsmittel oder Loesungsmittelgemisch wie Methanol, Ethanol, Dioxan, Tetrahydrofuran, Chloroform oder Dimethylformamid in Gegenwart einer Saeure, die auch selber als Loesungsmittel dienen kann, wie Schwefelsaeure, Salzsaeure, Salpetersaeure, Polyphosphorsaeure oder Flußsaeure oder mit einr Lewissaeure wie Aluminiumchlorid, Zinn(IV)chlorid, Zinkchlorid, Bortrifluorid oder Titan(IV)chlorid bei Temperaturen zwischen -20 °C und 50 °C durchgefuehrt;

l) fuer die Umsetzung von Verbindungen der allgemeinen Formel Id

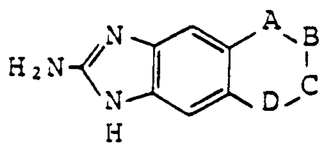

(Id),

in der A, B, C und D die angegebene Bedeutung haben, mit Verbindungen der allgemeinen Formel X,

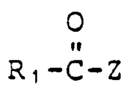

(X)

in der $R_1$ die angegebene Bedeutung hat und Z zusammen mit der C=O-Gruppe eine Carbonsaeure, Carbonsaeurehalogenid, Ester oder ein anderes aktiviertes Carbonsaeurederivat oder Anhydrid bedeutet, zu Verbindungen der allgemeinen Formel I, in der $R_1$, A, B, C und D die genannten Bedeutungen haben und X eine Amidgruppe -CONH-darstellt.

Die Umsetzung wird in einem Loesungsmittel oder Loesungsmittelgemisch wie Ethanol, Isopropanol, Eisessig, Benzol, Chlorbenzol, Glycol, Diethylenglycoldimethylether, Methylenchlorid, Pyridin, Sulfolan oder Dimethylformamid gegebenenfalls unter Zusatz einen Hilfsbase wie Triethylamin, Natriumcarbonat oder Natriumhydroxid oder eines Kondensationsmittels wie Phosphoroxychlorid, Thionylchlorid oder Polyphosphorsaeure bei Temperaturen zwischen -20 °C und 250 °C, vorzugsweise zwischen 0 °C und dem Siedepunkt des Loesungsmittels, durchgefuehrt.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt, weisen die neuen Verbindungen der allgemeinen Formel I, deren Tautomere optisch aktiven Verbindungen und Racemate und deren physiologisch vertraegliche Saeureadditionssalze ueberlegene pharmakologische Eigenschaften auf, insbesondere hemmen sie die Aggregation von Thrombozyten und Erythrozyten und/oder wirken blutdrucksenkend und/oder steigern die Herzkraft.

Zur Herstellung von Arzneimitteln werden die Substanzen der allgemeinen Formel I in an sich bekannnter Weise mit geeigneten pharmazeutischen Traegersubstanzen, Aroma-, Geschmacks-und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Pufferenthaelt.

Derartige Zusaetze sind z.B. Tartrat- und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Wieselsaeuren, hochmolekulare Fettsaeuren (wie Staerinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 1 -500 mg pro Tag bezogen auf 75 kg Koerpergewicht appliziert. Bevorzugt ist es, 2-3 mal pro Tag 1-2 Tabletten mit einem Wirkstoffgehalt von 1-200 mg zu verabreichen. Die Tabletten koennen auch retardiert sein, wodurch nur noch 1mal pro Tag 1-2 Tabletten mit 1 -500 mg Wirkstoff gegeben werden muß. Der Wirkstoff kann auch durch Injektion 1-8mal pro Tag. bzw. durch Dauerinfusion gegeben werden, wobei Mengen von 0,1-200 mg/Tag normalerweise ausreichen.

Besonders bevorzugt im Sinne der vorliegenden Erfindung sind außer den in den Beispielen genannten Verbindungen die folgenden und deren Tautomere:

7,7-Dimethyl-2-(4-methoxyphenyl)-5,6,7,8-tetrahydro-1H-imidazo[4,5-g]chinolin-6-on
7,7-Dimethyl-2-(4-methylphenyl)-5,6,7,8-tetrahydro-1H-imidazo [4,5-g]chinolin-6-on
7,7-Dimethyl-2-(4-trifluormethylphenyl)-5,6,7,8-tetrahydro-1H-imidazo[4,5-g]chinolin-6-on
7,7-Dimethyl-2-(2-methoxy-4-methylphenyl)-5,6,7,8-tetrahydro-1H-imidazo [4,5-g]chinolin-6-on
7,7-Dimethyl-2-(2-methoxy-4-methylmercaptophenyl)-5,6,7,8-tetrahydro-1H-imidazo[4,5-g]chinolin-6-on
7,7-Dimethyl-2-(2-methoxy-4-methylsulfinylphenyl)-5,6,7,8-tetrahydro-1H-imidazo[4,5-g]chinolin-6-on
2'-(4-Methoxyphenyl)spiro[cyclopentan-1,7'-5',6',7',8'-tetrahydro-1'H-imidazol[4',5'-g]chinolin]-6'-on
2'-(2-Methoxy-4-methylsulfinylphenyl)spiro[cyclopentan-1,7'-5',6',7',8'-tetrahydro-1'H-imidazo[4',5'-g]-chinolin]-6'-on
8,8-Dimethyl-2,3,5,6,7,8-hexahydro-1H-imidazo[4,5-g]chinolin-2,6-dion
8,8-Dimethyl-2-amino-5,6,7,8-tetrahydro-1H-imidazo [4,5-g]chinolin-6-on
8,8-Dimethyl-2-(1-propen-1-yl)-5,6,7,8-tetrahydro-1H-imidazo[4,5-g]chinolin-6-on
8,8-Dimethyl-2-(1-penten-1-yl)-5,6,7,8-tetrahydro-1H-imidazo[4,5-g]chinolin-6-on
8,8-Dimethyl-2-(1,3-pentadien-1-yl)-5,6,7,8-tetrahydro-1H-imidazo[4,5-g]chinolin-6-on
8,8-Dimethyl-2-(2-ethoxycarbonylmethyloxyphenyl)-5,6,7,8-tetrahydro-1H-imidazo[4,5-g]chinolin-6-on
8,8-Dimethyl-2-(2-methylmercaptophenyl)-5,6,7,8-tetrahydro-1H-imidazo[4,5-g]chinolin-6-on
8,8-Dimethyl-2-(4-acetylaminophenyl)-5,6,7,8-tetrahydro-1H-imidazo [4,5-g]chinolin-6-on
8,8-Dimethyl-2-(4-aminosulfonylphenyl)-5,6,7,8-tetrahydro-1H-imidazo[4,5-g]chinolin-6-on
8,8-Dimethyl-2-(4-morpholinosulfonylphenyl)-5,6,7,8-tetrahydro-1H-imidazo[4,5-g]chinolin-6-on
8,8-Dimethyl-2-(2-methoxy-4-methylsulfonylphenyl)-5,6,7,8-tetrahydro-1H-imidazo[4,5-g]chinolin-6-on
8,8-Dimethyl-2-(2-pyrrolyl)-5,6,7,8-tetrahydro-1H-imidazo[4,5-g]chinolin-6-on
8,8-Dimethyl-2-(1-imidazolyl)-5,6,7,8-tetrahydro-1H-imidazo[4,5-g]chinolin-6-on
8,8-Dimethyl-2-(2-methyl-4-oxazolyl)-5,6,7,8-tetrahydro-1H-imidazo[4,5-g]chinolin-6-on
8,8-Dimethyl-2-(5-isoxazolyl)-5,6,7,8-tetrahydro-1H-imidazo[4,5-g]chinolin-6-on
8,8-Dimethyl-2-(4-thiazolyl)-5,6,7,8-tetrahydro-1H-imidazo[4,5-g]chinolin-6-on
8,8-Dimethyl-2-(1,2,4-triazol-5-yl)-5,6,7,8-tetrahydro-1H-imidazo[4,5-g]chinolin-6-on
8,8-Dimethyl-2-(1,2,5-thiadiazol-3-yl)-5,6,7,8-tetrahydro-1H-imidazo[4,5-g]chinolin-6-on
8,8-Dimethyl-2-(1,2,3-thiadiazol-4-yl)-5,6,7,8-tetrahydro-1H-imidazo[4,5-g]chinolin-6-on
8,8-Dimethyl-2-(1,2,3-thiadiazol-5-yl)-5,6,7,8-tetrahydro-1H-imidazo[4,5-g]chinolin-6-on
8,8-Dimethyl-2-(2-methylmercapto-1,3,4-oxadiazol-5-yl)-5,6,7,8-tetrahydro-1H-imidazo[4,5-g]chinolin-6-on
8,8-Dimethyl-2-(2-pyridyl)-5,6,7,8-tetrahydro-1H-imidazo[4,5-g]chinolin-6-on
8,8-Dimethyl-2-(4-thiomorpholinyl)-5,6,7,8-tetrahydro-1H-imidazo[4,5-g]chinolin-6-on
8,8-Dimethyl-2-(2-naphthyl)-5,6,7,8-tetrahydro-1H-imidazo[4,5-g]chinolin-6-on
8,8-Dimethyl-2-(2-benzofuranyl)-5,6,7,8-tetrahydro-1H-imidazo[4,5-g]chinolin-6-on
8,8-Dimethyl-2-(3-chinolinyl)-5,6,7,8-tetrahydro-1H-imidazo[4,5-g]chinolin-6-on
2′-(4-Methoxyphenyl)spiro[cyclopentan-1,8′-5′,6′,7′,8′-tetrahydro-1H-imidazo[4′,5′-g]chinolin]-6′-on
2′-(4-Methylphenyl)spiro[cyclopentan-1,8′-5′,6′,7′,8′-tetrahydro-1′H-imidazo[4′,5′-g]chinolin]-6′-on
2′-(4-Trifluormethylphenyl)spiro[cyclopentan-1,8′-5′,6′,7′, 8′-tetrahydro-1′H-imidazo[4′,5′-g]chinolin]-6′-on
2′-(2-Methoxy-4-methylmercaptophenyl)spiro[cyclopentan]1,8′-5′,6′,7′,8′-tetrahydro-1′H-imidazo[4′,5′-g]-chinolin]-6′-on
2′-(2-Methoxy-4-methylsulfonylphenyl)spiro[cyclopentan-1,8′-5′,6′,7′,8′-tetrahydro-1′H-imidazo[4′,5′-g]-chinolin]-6′-on
2′-(2-Methoxy-4-methylsulfonyloxyphenyl)spiro[cyclopentan-1,8′-5′,6′,7′,8′-tetrahydro-1′H-imidazo[4′,5′-g]-

chinolin]-6′-on
2′-(Chinolin-4-yl)spiro[cyclopentan-1,8′-5′,6′,7′,8′-tetrahydro-1′H-imidazo[4′,5′-g]chinolin]-6′-on
2′-(3-Methyl-pyrazol-5-yl__spiro[cyclopentan-1,8′-5′,6′,7′, 8′-tetrahydro-1′H-imidazo[4′,5′-g]chinolin]-6′-on
2′-(4-methoxyphenyl)spiro[cyclohexan-1,8′-5′,6′,7′,8′-tetrahydro-1′H-imidazo[4′-5′-g]chinolin]-6′-on
8,8-Dimethyl-2-(4-methoxyphenyl)-5,6,7,8-tetrahydro-1H-imidazo[4,5-g]isochinolin-5-on
8,8-Dimethyl-2-(4-trifluormethylphenyl)-5,6,7,8-tetrahydro-1H-imidazo[4,5-g]isochinolin-5-on
8,8-Dimethyl-2-(2-methoxy-4-methylphenyl)-5,6,7,8-tetrahydro-1H-imidazo[4,5-g]isochinolin-5-on
8,8-Dimethyl-2-(2-methoxy-4-methylmercaptophenyl)-5,6,7,8-tetrahydro-1H-imidazo[4,5-g]isochinolin-5-on
8,8-Dimethyl-2-(2-methoxy-4-methylsulfinylphenyl)-5,6,7,8-tetrahydro-1H-imidazo[4,5-g]isochinolin-5-on
8,8-Dimethyl-2-(2-methoxy-4-methylsulfonylphenyl)-5,6, 7,8-tetrahydro-1H-imidazo[4,5-g]isochinolin-5-on
8,8-Dimethyl-2-(2-methoxy-4-methylsulfonylphenyl)-5,6,7,8-tetrahydro-1H-imidazo[4,5-g]isochinolin-5-on
8,8-Dimethyl-2-(chinolin-4-yl)-5,6,7,8-tetrahydro-1H-imidazo[4,5-g]isochinolin-5-on
8,8-Dimethyl-2-(3-methyl-pyrazol-5-yl)-5,6,7,8-tetrahydro-1H-imidazo[4,5-g]isochinolin-5-on
2′-(4-Methoxyphenyl)spiro[cyclopentan-1,8′-5′,6′,7′,8′-tetrahydro-1′H-imidazo[4′,5′-g]isochinolin]-5′-on
2′-(4-Methylphenyl)spiro[cyclopentan-1,8′-5′,6′,7′,8′-tetrahydro-1′H-imidazo[4′,5′-g]isochinolin]-5′-on
2′-(4-Trifluormethylphenyl)spiro[cyclopentan-1,8′-5′,6′,7′,8′-tetrahydro-1′H-imidazo[4′,5′-g]isochinolin]-5′-on
2′-(2-Methoxy-4-methylmercaptophenyl)spiro[cyclopentan-1,8′-5′,6′,7′,8′-tetrahydro-1′H-imidazo[4′,5′-]isochinolin]-5′-on
2′-(2-Methoxy-4-methylsulfinylphenyl)spiro[cyclopentan-1,8′-5′,6′,7′,8′-tetrahydro-1′H-imidazo[4′,5′-g]-isochinolin]-5′-on
2′-(2-Methoxy-4-methylsulfonylphenyl)spiro[cyclopentan-1,8′-5′,6′,7′,8′-tetrahydro-1′H-imidazo[4′,5′-g]-isochinolin]-5′-on
2′-(2-Methoxy-4-methylsulfonyloxyphenyl)spiro[cyclopentan-1,8′-5′,6′,7′,8′-tetrahydro-1′H-imidazo[4′,5′-g]-isochinolin]-5′-on
2′-(Chinolin-4-yl)spiro[cyclopentan-1,8′-5′,6′,7′,8′-tetrahydro-1′H-imidazo[4′,5′-g]isochinolin]-5′-on
2′-(3-Methyl-pyrazol-5-yl)spiro[cyclopentan-1,8′-5′,6′,7′8′-tetrahydro-1′H-imidazo[4′,5′-g]isochinolin]-5′-on
7,8-Dimethyl-2-(4-methoxyphenyl)-5,6-dihydro-1H-imidazo[4,5-g]isochinolin-5-on
7,8-Dimethyl-2-(2-methoxy-4-methylmercaptophenyl)-5,6-dihydro-1H-imidazo[4,5-g]isochinolin-5-on
7,8-Dimethyl-2-(2-methoxy-4-methylsulfinylphenyl)-5,6-dihydro-1H-imidazo[4,5-g]isochinolin-5-on
7,8-Dimethyl-2-(2-methoxy-4-methylsulfonylphenyl)-5,6-dihydro-1H-imidazo[4,5-g]isochinolin-5-on
7,8-Dimethyl-2-(2-methoxy-4-methylsulfonyloxyphenyl)-5,6-dihydro-1H-imidazo[4,5-g]isochinolin-5-on
8,8-Dimethyl-2-(4-methoxyphenyl)-5,6,7,8-tetrahydro-1H-imidazo[4,5-g]isochinolin-5,7-dion
8,8-Dimethyl-2-(2-methoxy-4-methylsulfinylphenyl)-5,6,7, 8-tetrahydro-1H-imidazo[4,5-g]isochinolin-5,7-dion
8,8-Dimethyl-2-(2-methoxy-4-methylsulfonylphenyl)-5,6,7, 8-tetrahydro-1H-imidazo[4,5-g]isochinolin-5,7-dion
2′-(4-Methoxyphenyl)spiro[cyclopentan-1,8′-5′,6′,7′,8′-tetrahydro-1′H-imidazo[4′,5′-g]isochinolin]-5′,7′-dion
2′-(2-Methoxy-4-methylsulfinylphenyl)spiro[cyclopentan-1,8′-5′,6′,7′,8′-tetrahydro-1′H-imidazo[4′,5′-g]-isochinolin ]-5′,7′-dion
2'-(2-Methoxy-4-methylsulfonylphenyl)spiro[cyclopentan-1,8'-5',6',7',8'-tetrahydro-1'H-imidazo[4',5'-g]-isochinolin]-5',7'-dion

## Beispiel 1

7,7-Dimethyl-2-(4-pyridyl)-5,6,7,8-tetrahydro-1H-imidazo[4,5-g]chinolin-6-on x 0.5 mol Isopropanol

3 g (8.8 mmol) 3,3-Dimethyl-6-nitro-7-isonicotinoylamino-1,2,3,4-tetrahydro-chinolin-2-on wurden in 300 ml Ethanol suspendiert, 0.3 g 10proz. Palladium/Kohle zugesetzt und hydriert. Nach Abtrennung des Katalysators wurde das Filtrat eingedampft und eine Stunde mit Eisessig bei 60 °C geruehrt. Nach Eindampfen, Neutralisieren und Umkristallisieren aus Isopropanol erhaelt man 1.9 g (74 %) der Titelverbindung vom Smp. >300 °C.

Das Ausgangsmaterial wurde wie folgt hergestellt:

a) 24.55 g (137 mmol) 2,2-Dimethyl-3-phenyl-propionsaeure wurden zunaechst unter Eiskuehlung mit 180 ml 96proz. $HNO_3$ versetzt und anschließend eine Stunde bei 100 °C gehalten. Aufarbeitung (Eis, $CH_2Cl_2$, $MgSO_4$) und Umkristallisieren aus Toluol/$CH_2Cl_2$ liefert 19.8 g (54 %) 2,2-Dimethyl-3-(2,4-dinitrophenyl)propionsaeure vom Smp. 120-122 °C.

b) 21.8 g (81 mmol) der obigen Saeure wurden in Ethanol mit katalytischen Mengen conc.$H_2SO_4$ verestert. Nach Aufarbeitung (Ether, $NaHCO_3$-Loesung, $MgSO_4$) wurden 23.8 g Ester als Oel erhalten.

c) 23.8 g (80 mmol) des obigen Esters wurden in Ethanol an 10proz. Palladium/Kohle hydriert. Nach Abtrennung des Katalysators und Eindampfen des Ethanols erhaelt man 12.9 g (85 %) 7-Amino-3,3-dimethyl-1,2,3,4-tetrahydrochinolin-2-on vom Smp. 232-35 °C.

d) 12.5 g (65 mmol) des obigen Amins wurden mit 12.9 g (72 mmol) Isonicotinsaeurechlorid x HCl in 400 ml $CH_2Cl_2$ und 19.9 ml Triethylamin acyliert. Aufarbeitung des eingedampften Rueckstandes erfolgte durch Behandeln mit Wasser und Umkristallisierung unter Zusatz von Kohle aus Isopropanol. Man erhaelt 15.4 g (79 %) 7-Isonicotinoylamino-3,3-dimethyl-1,2,3,4-tetrahydrochinolin-2-on vom Smp. 242-44 °C.

e) 15.4 g (52 mmol) des obigen Amids wurden bei -30 °C in 300 ml 96 % $HNO_3$ nitriert. Nach Aufarbeitung (Eis, Neutralisieren, Absaugen) wurde der Rueckstand ueber Kieselgel (Laufmittel: $CH_2Cl_2$/1 % $CH_3OH$) gereinigt. Man erhaelt 6 g (33 %) 6-Nitro-7-isonicotinoylamino-3,3-dimethyl-1,2,3,4-tetrahydrochinolin-2-on vom Smp. 145-48 °C.

Vergleichsbeispiel 2

2-(4-Pyridyl)-7,8-dihydro-1H-imidazo[4,5-g]chinazolin-8-on x $H_2O$ x HCl

2.9 g (1.64 mmol) 6,7-Diamino-3,4-dihydro-chinazolin-4-on (J.Org.Chem. 40, 360, 1975) wurden in 145 ml DMF mit 4.35 g (2.44 mmol) Isonicotinsaeurechlorid x HCl und 7.4 g Triethylamin acyliert. Nach 4 Stunden wurde der Ansatz eingedampft, der Rueckstand mit Wasser versetzt, abgesaugt und der Rueckstand mit 250 ml Ethanol heiß ausgeruehrt und abgesaugt. Die Kristalle wurden anschließend 24 Stunden mit 75 ml Ethanol und 120 ml conc. HCl zum Rueckfluß erhitzt, abgesaugt, in 2 N NaOH geloest, gekohlt, mit conc. HCl angesaeuert und abgesaugt. Man erhaelt 1.8 g der Titelverbindung als Hydrochlorid vom Smp. >300 °C.

Vergleichsbeispiel 2a

2-(4-Methoxyphenyl)-7,8-dihydro-1H-imidazo[4,5-g]-chinazolin-8-on

In analoger Weise wie in Beispiel 20 beschrieben, erhält man aus 4-Methoxybenzaldehyd, Natriumhydrogensulfit und 6,7-Diamino-3,4-dihydro-chinazolin-4-on (Bsp. 2) nach Auskochen mit Methanol die Titelverbindung in 82 %iger Ausbeute mit einem Schmelzpunkt >300 °C.

Vergleichsbeispiel 3

2-(4-Pyridyl)-5,6-dihydro-1H-imidazo[4,5-g]chinoxalin-6-on

3 g (11.8 mmol) 2-(4-Pyridyl)-5-amino-6-nitro-benzimidazol wurden in 500 ml Methanol an 0.8 g $PtO_2$ hydriert. Nach Abtrennung des Katalysators wurde das Filtrat mit 10 ml Glyoxylsaeureechylester versetzt, und 3 Stunden unter Stickstoff bei 60 °C gekocht. Nach Einengen des Methanols wird warm abgesaugt und die Kristalle unter Zusatz von Kohle nochmals aus Methanol umkristallisiert. Man erhaelt 1.5 g (48.5 %) der Titelverbindung vom Smp. >320 °C.

Das Ausgangsmaterial wurde wie folgt hergestellt:

a) 4.6 g (27.4 mmol) 1,2,4-Triamino-5-nitrobenzol (Org. Synth. 40, 96) wurde in 60 ml Methylenchlorid suspendiert und mit 6.55 g Isonicotinsaeurechlorid x HCl und 11.4 ml Triethylamin acyliert. Anschließend wurde das Methylenchlorid abgedampft, der Rueckstand mit Wasser durchgearbeitet und der noch feuchte Rueckstand mit 250 ml Ethanol und 40 ml konz. Salzsaeure 20 Stunden refluxiert. Der eingedampfte Rueckstand wurde in 250 ml Wasser weitgehend geloest, neutralisiert, abgesaugt, mit Ethanol aufgekocht, eingeengt und das Kristallisat abgesaugt. Man erhaelt 5.5 g (79 %) 2-(4-Pyridyl)-5-amino-6-nitrobenzimidazol.

Beispiel 4

8,8-Dimethyl-2-(4-methoxyphenyl)-5,6,7,8-tetrahydro-1H-imidazo[4,5-g]chinolin-6-on

6.8 g (18.4 mmol) 4,4-Dimethyl-6-(4-methoxybenzoylamino)-7-nitro-1,2,3,4-tetrahydro-chinolin-2-on wurden in 200 ml Ethanol an 0.6 g 10proz. Palladium auf Kohle hydriert. Nach Abtrennen des Katalysators wurde das Filtrat mit 15 ml konz. Salzsaeure versetzt und 20 Stunden Rueckfluß gekocht. Der eingedampfte Rueckstand wurde mit Wasser und Ammoniak versetzt, mit Methylenchlorid/Methanol 20:1 extrahiert, ueber Kieselgel (Laufmittel: Methylenchlorid/mit Ammoniak gesaettigtes Methanol 20:1) gereinigt und aus Ethanol umkristallisiert. Man erhaelt 3.5 g (60 %) der Titelverbindung vom Smp. 235-38 °C.

Das Ausgangsmaterial wurde wie folgt hergestellt:

a) 29 g (165 mmol) 3,3-Dimethylacrylsaeureanilid wurden mit 58 g $AlCl_3$ und 70 g NaCl gut durchmischt und nach Abklingen der exothermen Reaktion 2 Stunden auf 100 °C erwaermt. Nach Zersetzen mit Eis, Extrahieren, Trocknen und Einengen wird das Oel mit wenig Ether ausgeruehrt und das Kristallisat abgesaugt. Man erhaelt 19.8 g (68 %) 4,4-Dimethyl-1,2,3,4-tetrahydro-chinolin-2-on vom Smp. 113-15 °C.

b) 24.7 g (140 mmol) des obigen Produktes wurden in 250 ml 80proz. $H_2SO_4$ vorgelegt und bei 5 °C eine Mischung aus 6.5 ml 96proz. $HNO_3$ und 30 ml 80proz. $H_2SO_4$ zugetropft. Nach 1 Stunde gießt man auf Eis, saugt ab, waescht mit Wasser und kristallisiert aus Isopropanol. Man erhaelt 24.6 g (80 %) 4,4-Dimethyl-6-nitro-1,2,3,4-tetrahydro-chinolin-2-on vom Smp. 202-04 °C.

c) 15.2 g (69 mmol) der obigen Substanz wurden in 300 ml Methanol an 1 g 10proz. Palladium auf Kohle hydriert. Nach Abtrennen des Katalysators wurde die Loesung des Amins mit 25 ml Essigsaeureanhydrid versetzt, langsam im Vakuum eingedampft, der Rueckstand mit Essigester digeriert und abgesaugt. Man erhaelt 15.1 g (94 %) 4,4-Dimethyl-6-acetylamino-1,2,3,4-tetrahydro-chinolin-2-on.

d) 15 g (65 mmol) des obigen Acetamids wurden in 100 ml 90proz. Schwefelsaeure bei 5 °C t opfenweise mit 3 ml 96proz. Salpetersaeure versetzt. Nach 30 Minuten bei 25 °C wurde auf Eis gegossen, abgesaugt und mit Wasser gewaschen. Man erhaelt 14.6 g (82 %) 4,4-Dimethyl-6-acetyl-amino-7-nitro-1,2,3,4-tetrahydro-chinolin-2-on vom Smp. 272-78 °C.

e) 14.2 g (51.3 mmol) der obigen Substanz wurden in 150 ml Ethanol und 30 ml konz. Salzsaeure 8 Stunden gekocht. Der Ansatz wurde auf das doppelte Volumen mit Wasser verduennt, abgesaugt und mit Wasser nachgewaschen. Man erhaelt 9.7 g (80 %) 4,4-Dimethyl-6-amino-7-nitro-1,2,3,4-tetrahydro-chinolin-2-on vom Smp. 313-17 °C.

f) 4.7 g (20 mmol) der obigen Substanzen wurden in 50 ml Pyridin unter Eiskuehlung mit 3.7 g (22 mmol) 4-Methoxybenzoylchlorid versetzt. Nach 2 Stunden bei 25 °C gießt man auf Eis, saeuert mit 2 N Salzsaeure an, extrahiert mit Methylenchlorid, trocknet und engt ein. Man erhaelt 6.9 g (93 %) 4,4-Dimethyl-6-(4-methoxybenzoylamino)-7-nitro-1,2,3,4-tetrahydro-chinolin-2-on vom Smp. 288-90 °C.

Beispiel 5

8,8-Dimethyl-2-(4-pyridyl)-5,6,7,8-tetrahydro-1H-imidazo[4,5-g]chinolin-6-on

Analog Beispiel 4 erhaelt man die Titelverbindung in 72 % Ausbeute vom Smp. 201-202 °C.

Beispiel 6

2-(4-Pyridyl)-5,6,7,8-tetrahydro-1H-imidazo[4,5-g]chinolin-6-on

2.4 g (7.7 mmol) 6-Isonicotinoylamino-7-nitro-1,2,3,4-tetrahydro-chinolin-2-on wurden in 100 ml Methanol an 0.4 g 10proz. Palladium auf Kohle hydriert. Nach Abtrennen des Katalysators wurde der eingedampfte Rueckstand mit 80 ml Ethanol und 15 ml konz. Salzsaeure 24 Stunden gekocht. Der eingedampfte Rueckstand wurde mit Wasser und Ammoniak behandelt, abgesaugt und aus Methanol umkristallisiert. Man erhaelt 1.3 g (62 %) der Titelverbindung vom Smp. oberhalb 300 °C.

Das Ausgangsmaterial wurde wie folgt hergestellt:

a) 37 g (250 mmol) 1,2,3,4-Tetrahydro-chinolin-2-on wurden in 100 ml konz. Schwefelsaeure bei 0-5 °C mit Nitriersaeure (17.3 g 96proz. $HNO_3$ + 30 g konz. $H_2SO_4$) nitriert. Nach dem Zutropfen wurde noch 5 Minuten nachgeruehrt, auf Eis gegossen, abgesaugt und gut mit Wasser gewaschen. Man erhaelt 30 g (62 %) 6-Nitro-1,2,3,4-tetrahydro-chinolin-2-on vom Smp. 202-204 °C.

b) 30 g (156 mmol) der Nitroverbindung wurden in 300 ml Methanol an 3 g 10proz. Palladium auf Kohle hydriert. Nach Abtrennen des Katalysators und Eindampfen des Loesungsmittels erhaelt man 25 g (98 %) 6-Amino-1,2,3, 4-tetrahydro-chinolin-2-on vom Smp. 178-81 °C.

c) 13.9 g (85.7 mmol) des Amins wurden mit 16.8 g (94.3 mmol) Isonicotinsaeurechlorid x HCl und 26.3 ml Triethylamin in 200 ml Methylenchlorid acyliert. Der eingedampfte Rueckstand wurde mit Wasser durchgearbeitet, abgesaugt und getrocknet. Man erhaelt 22 g (98 %) 6-Isonicotinoylamino-1,2,3,4-tetrahydro-chinolin-2-on vom Smp. 232-34 °C.

d) 22.7 g (85 mmol) des obigen Amids wurden langsam bei 5 °C in 120 ml 65proz. Salpetersaeure eingetragen. Bei 25 °C eine Stunde weitergeruehrt, auf Eis gegossen und abgesaugt. Der Rueckstand wurde ueber Kieselgel (Laufmittel: $CH_2Cl_2$/mit Ammoniak gesaettigtes $CH_3OH$, (95:5) gereinigt. Man erhaelt 2.4 g (10 %) 6-Isonicotinoylamino-7-nitro-1,2,3,4-tetrahydro-chinolin-2-on vom Smp. 285-88 °C.

Beispiel 7

8,8-Dimethyl-2-(4-pyridyl)-5,6,7,8-tetrahydro-1H-imidazo[4,5-g]isochinolin-5-on

5 g (20.4 mmol) 4,4-Dimethyl-6-nitro-7-amino-1,2,3,4-tetrahydro-isochinolin-1-on wurden in 120 ml Methanol an 0.5 g 10proz. Palladium auf Kohle hydriert. Nach Abtrennen des Katalysators wurde der eingedampfte Rueckstand (4.3 g) in 150 ml Methylenchlorid und 8.4 ml Triethylamin mit 5.4 g Isonicotinsaeurechlorid x HCl acyliert.

Nach 1 Stunde wird der eingedampfte Rueckstand mit Wasser durchgearbeitet und abgesaugt. Der feuchte Rueckstand wird anschließend in 100 ml Ethanol und 20 ml konz. Salzsaeure 24 Stunden gekocht, eingedampft, mit Wasser versetzt und neutralisiert. Nach Saeulenchromatographie (Kieselgel, Laufmittel: Methylenchlorid/mit Ammoniak gesaettigtes Methanol, 15:1) und Auskochen erhaelt man 2.3 g (41 %) der Titelverbindung vom Smp. oberhalb 300 °C.

Das Ausgangsmaterial wurde wie folgt hergestellt:

a) 44.5 g (240.5 mmol) 4,4-Dimethyl-1,2,3,4-tetrahydro-isochinolin-1-on (J.Het.Chem. 7, 615, 1970) in 200 ml konz. Schwefelsaeure bei -10-0 °C wurden mit 10.5 ml 96proz. Salpetersaeure tropfenweise nitriert. Anschließend gießt man auf Eis, saugt ab, waescht mit Wasser nach und reinigt den getrockneten Rueckstand ueber Kieselgel (Laufmittel: $CH_2Cl_2$/Essigester, 2:1). Man erhaelt 47 g (85 %) 4,4-Dimethyl-7-nitro-1,2,3,4-tetrahydro-isochinolin-1-on vom Smp. 227-30 °C.

b) 47 g (204 mmol) der obigen Substanz wurden in 1000 ml Methanol an 3 g 10proz. Palladium auf Kohle hydriert, anschließend vom Katalysator abgetrennt und der eingedampfte Rueckstand in 300 ml Essigester suspendiert und mit 30 ml Essigsaeureanhydrid versetzt. Nach 30 Minuten wird abgesaugt und mit Essigester gewaschen. Man erhaelt 46.7 g (94 %) 4,4-Dimethyl-7-acetylamino-1,2,3,4-tetrahydro-isochinolin-1-on vom Smp. 250-51 °C.

c) 45 g (186 mmol) der obigen Substanz wurden in 300 ml konz. Schwefelsaeure bei 0 °C durch Zutropfen von Nitriersaeure (8.5 ml 96proz. $HNO_3$ + 30 ml konz. $H_2SO_4$) nitriert. Nach beendeter Zugabe wurde noch 3 Stunden bei 25 °C weitergeruehrt, auf Eis gegossen, abgesaugt und aus 400 ml Eisessig umkristallisiert. Das Kristallisat enthaelt das falsche Isomere (24 g). Das Filtrat wurde eingedampft und mit 200 ml Ethanol und 40 ml konz. Salzsaeure 20 Stunden gekocht, eingeengt mit Wasser versetzt, neutralisiert, mit Methylenchlorid extrahiert, getrocknet und eingedampft. Der Rueckstand wurde durch Saeulenchromatographie (Kieselgel, Laufmittel: Essigester/Methylenchlorid, 1:10 bis 1:1) gereinigt. Man erhaelt 11 g (25 %) 4,4-Dimethyl-6-nitro-7-amino-1,2,3,4-tetrahydro-isochinolin-1-on vom Smp. 231-34 °C.

Beispiel 8

8,8-Dimethyl-2-(4-pyridyl)-5,6,7,8-tetrahydro-1H-imidazo[4,5-g]isochinolin-5,7-dion

27 g (82 mmol) 4,4-Dimethyl-6-nitro-7-amino-8-brom-1,2,3,4-tetrahydro-isochinolin-1,3-dion wurden in 300 ml Methanol an 2 g 10proz. Palladium auf Kohle hydriert. Nach Abtrennen des Katalysators wird das Filtrat eingedampft, der Rueckstand mit 500 ml Methylenchlorid und 50 ml Triethylamin versetzt und unter Kuehlung 24 g Isonicotinsaeurechlorid x HCl langsam eingetragen. Nach 4 Stunden wurde das Methylenchlorid abgedampft, mit Wasser durchgearbeitet, abgesaugt und der feuchte Rueckstand in 500 ml Ethanol und 200 ml konz. Salzsaeure 20 Stunden zum Rueckfluß erhitzt.

Der eingedampfte Rueckstand wurde anschließend mit Wasser versetzt, neutralisiert und durch Saeulenchromatographie (Kieselgel, Laufmittel: Methylenchlorid/mit Ammoniak gesaettigtes Methanol, 10:1) gereinigt. Nach Umkristallisieren aus Ethanol/Wasser erhielt man 12.2 g (51 %) der Titelverbindung vom Smp. oberhalb 300 °C.

Das Ausgangsmaterial wurde wie folgt hergestellt:

a) 45 g (184 mmol) 4,4-Dimethyl-7-acetylamino-1,2,3,4-tetrahydro-isochinolin-1,3-dion wurden in 165 ml konz. Schwefelsaeure und 17 ml Wasser geloest, 33 g Silbersulfat zugesetzt und unter Eiskuehlung 9.5 ml Brom zugetropft, anschließend 30 Minuten bei 25 °C weitergeruehrt, auf Eis gegossen und abgesaugt. Der Rueckstand wurde mit Methylenchlorid/Methanol gewaschen, das Filtrat eingedampft, mit Ethanol digeriert und abgesaugt. Man erhaelt 42 g (95 %) 4,4-Dimethyl-7-acetylamino-8-brom-1,2,3,4-tetrahydro-isochinolin-1,3-dion.

b) 42 g (130 mmol) der obigen Substanz wurden bei 5 °C in eine Mischung von 400 ml konz. Schwefelsaeure und 80 ml 96proz. Salpetersaeure eingetragen. Man ruehrt 45 Minuten bei 15 °C, gießt auf Eis, saugt ab und waescht mit Wasser. Man erhaelt 37 g (77 %) 4,4-Dimethyl-6-nitro-7-acetylamino-8-brom-1,2,3,4-tetrahydro-isochinolin-1,3-dion.

c) 37 g (100 mmol) der obigen Verbindung wurden durch 20 Stunden Rueckfluß in 350 ml Ethanol und 175 ml konz. Salzsaeure entacetyliert. Nach dem Abkuehlen gießt man auf Eis, saugt ab und waescht mit Wasser. Man erhaelt 27.4 g (84 %) 4,4-Dimethyl-6-nitro-7-amino-8-brom-1,2,3,4-tetrahydro-isochinolin-1,3-dion vom Smp. 218-21 °C.

Beispiel 9

8,8-Dimethyl-7-hydroxy-2-(4-pyridyl)-5,6,7,8-tetrahydro-1H-imidazo[4,5-g]isochinolin-5-on

5 g (17 mmol) der in Beispiel 8 erhaltenen Verbindung werden in 250 ml Dioxan, 250 ml Methanol und 25 ml Wasser suspendiert. Unter Kuehlung mit Wasser gibt man Natriumborhydrid in 0.5 g Portion zu. Nach jeder Zugabe wird der Reaktionsverlauf chromatographisch verfolgt. Wenn die Substanz gerade in Loesung ist (Umsatz 70 %) wird die Reaktion abgebrochen. Das Produkt wird durch Zugabe von einem Liter Wasser gefaellt und abgedampft. Man erhaelt 3.8 g (76 %) der Titelverbindung vom Smp. 260-68 °C.

Beispiel 10

7,8-Dimethyl-2-(4-pyridyl)-5,6-dihydro-1H-imidazo[4,5-g]isochinolin-5-on

3.5 g (11.4 mmol) der in Beispiel 9 erhaltenen Verbindung werden unter Kuehlung in 35 ml konz. Schwefelsaeure eingetragen, 4 Stunden bei 25 °C geruehrt, auf Eis gegossen, neutralisiert und abgesaugt. Der Rueckstand wurde durch Saeulenchromatographie an Kieselgel (Laufmittel: $CH_2Cl_2$/mitAmmoniak gesaettigtes Methanol, 10:1 bis 5:1) gereinigt und die Titelverbindung nochmals in Methanol digeriert und abgesaugt. Man erhaelt 1.8 g (55 %) vom Smp. oberhalb 300 °C.

Vergleichsbeispiel 11

2-(4-Pyridyl)-5,6,7,8-tetrahydro-1H-imidazo[4,5-g]-chinoxalin-6,7-dion

Analog Beispiel 3 erhaelt man aus 2-(4-Pyridyl)-5-amino-6-nitro-benzimidazol durch Hydrierung und Umsetzung mit Oxalsaeurediethylester in 45 % Ausbeute die Titelverbindung vom Smp. oberhalb 300 °C.

Vergleichsbeispiel 12

5,8-Dimethyl-2-(4-pyridyl)-5,6,7,8-tetrahydro-1H-imidazo[4,5-g]chinoxalin-6,7-dion x HCl x $H_2O$

2.8 g (10 mmol) 1,4-Dimethyl-6,7-dinitro-1,2,3,4-tetrahydrochinoxalin-2,3-dion (J.Chem.Soc. 1170, 1962) wurden in 80 ml Ethanol an 0.28 g $PtO_2$ bei 5 bar und 40 °C 16 Stunden hydriert. Nach Abtrennen des Katalysators wurde dem Filtrat 1.07 g (10 mmol) Pyridin-4-aldehyd und 0.19 g (1 mmol) p-Toluolsulfonsaeure-Hydrat zugesetzt und 2 Stunden unter Luftdurchleiten erhitzt. Nach Einengen und Zusatz von ethanolischer Salzsaeure erhaelt man 1.1 g (36 %) der Titelverbindung vom Smp. 270 °C (Zers.).

Beispiel 13

8,8-Dimethyl-2-(3-chlorphenyl)-5,6,7,8-tetrahydro-1H-imidazo[4,5-g]chinolin-6-on

a) 50 g (0.21 mol) 4,4-Dimethyl-6-amino-7-nitro-1,2,3,4-tetrahydrochinolin-2-on (Bsp. 4 e) wurden in 3000 ml Methanol an 5 g Palladium auf Kohle (10proz.) hydriert. Nach Abtrennen des Katalysators und Eindampfen des Lösungsmittels erhält man nach Kristallisation aus Ethanol 35.2 g (81 %) 4,4-Dimethyl-6,7-diamino-1,2,3,4-tetrahydrochinolin-2-on vom Schmp. 192-94 °C.

b) 1 g (4.8 mmol) des obigen Diamins wurden mit 0.92 g (5.8 mmol) 3-Chlorbenzoesäure in 10 ml Polyphosphorsäure bei 160 °C für 2 Std. gerührt. Nach Verdünnen mit Eis/Wasser wurde abgesaugt, der Rückstand nochmals in Wasser suspendiert, mit 2 N Ammoniak neutralisiert und der abgesaugte Rückstand aus Ethanol umkristallisiert. Man erhält 1.2 g (76 %) der Titelverbindung vom Schmp. 190-93 °C.

Beispiel 14

In analoger Weise wie in Beispiel 13 beschrieben erhält man:

| | Bezeichnung | Ausb. % | Schmelzpunkt °C (Lsg.mittel) |
|---|---|---|---|
| a) | 8,8-Dimethyl-2-(4-chinolinyl)-5,6,7,8-tetrahydro-1H-imidazo[4-,5-g]chinolin-6-on als Dihydrochlorid | 60 | 265-68 $(CH_3)_2CHOH$ |
| b) | 8,8-Dimethyl-2-(4-methylphenyl)-5,6,7,8-tetrahydro-1H-imidazo[4,5-g]chinolin-6-on | 84 | 205-07 $CH_3OH$ |
| c) | 8,8-Dimethyl-2-(2-methoxyphenyl)-5,6,7,8-tetrahydro-1H-imidazo[4,5-g]chinolin-6-on | 25 | 240-42 $CH_3COOC_2H_5$ |
| d) | 8,8-Dimethyl-2-(4-pyridazinyl)-5,6,7,8-tetrahydro-1H-imidazo[-4,5-g]chinolin-6-on | 42 | >300 $CH_3OH$ |
| e) | 8,8-Dimethyl-2-(2-phenylethen-1-yl)-5,6,7,8-tetrahydro-1H-imidazo[4,5-g] chinolin-6-on | 15 | >300 $CH_3COOC_2H_5$ |
| f) | 8,8-Dimethyl-2-(1-hexyl)-5,6,7,8-tetrahydro-1H-imidazo[4,5-g-]chinolin-6-on | 62 | 214-15 $CH_3COOC_2H_5$ |
| g) | 8,8-Dimethyl-2-(4-chlorphenyl)-5,6,7,8-tetrahydro-1H-imidazo[4,5-g]chinolin-6-on | 48 | >300 $CH_3OH$ |

Beispiel 15

8,8-Dimethyl-2-(4-methoxybenzoylamino)-5,6,7,8-tetrahydro-1H-imidazo[4,5-g]chinolin-6-on

a) 3 g (14.6 mmol) 4,4-Dimethyl-6,7-diamino-1,2,3,4-tetrahydrochinolin-2-on (Bsp. 13 a) wurden in 100 ml Ethanol gelöst, mit 1.7 g (16.1 mmol) Bromcyan versetzt und 5 Std. bei 25 °C gerührt. Der eingedampfte Rückstand wurde nochmals in EtOH gelöst, gekühlt, auf ca. 10 ml eingedampft, Aceton zugesetzt und abgesaugt. Man erhält 4.25 g (94 %) 8,8-Dimethyl-2-amino-5,6,7,8-tetrahydro-1H-imidazo[4,5-g]chinolin-6-on als Hydrobromid vom Schmp. >300 °C.
b) 4.0 g (12.8 mmol) des obigen Hydrobromids wurden mit 3.3 g (19.2 mmol) 4-Methoxybenzoylchlorid in 80 ml Pyridin für 8 Std. bei 50 °C gerührt. Der eingedampfte Rückstand wurde in Wasser suspendiert, mit 2 N Ammoniak alkalisch gestellt und der Rückstand aus Methanol umkristallisiert. Man erhält 3 g (64 %) der Titelverbindung vom Schmp. >300 °C.

Beispiel 16

In analoger Weise wie in Beispiel 15 beschrieben erhält man:

| | Bezeichnung | Ausb. % | Schmelzpunkt °C (Lsg.mittel) |
|---|---|---|---|
| a) | 8,8-Dimethyl-2-(4-pyridyl-carbonylamino)-5,6,7,8-tetrahydro--1H-imidazo[4,5-g]chinolin-6-on | 80 | 230-33 $CH_3OH$ |

Beispiel 17

8,8-Dimethyl-2-hydroxy-5,6,7,8-tetrahydro-1H-imidazo[4,5-g]chinolin-6-on

2 g (9.7 mmol) 4,4-Dimethyl-6,7-diamino-1,2,3,4-tetrahydrochinolin-2-on(Bsp. 13 a) wurden in 50 ml 2 N Salzsäure gelöst und 1 Std. Phosgen eingeleitet. Der abgesaugte Rückstand wurde aus Methanol umkristallisiert. Man erhält 1.6 g (72 %) der Titelverbindung vom Schmp. >300 °C.

Beispiel 18

8,8-Dimethyl-2-[4-(1H-imidazol-1-yl)phenyl]-5,6,7,8-tetrahydro-1H-imidazo[4,5-g]chinolin-6-on

2 g (9.7 mmol) 4,4-Dimethyl-6,7-diamino-1,2,3,4-tetrahydro-chinolin-2-on (Bsp. 13 a), 1.67 g (9.7 mmol) 4-(1H-Imidazol-1yl)benzaldehyd, 0.17 g p-Toluolsulfonsäure und 120 ml Ethanol wurden zusammengegeben und 3 Std. unter Luftdurchleiten zum Rückfluß erhitzt. Der eingedampfte Rückstand wurde in Wasser suspendiert, mit 2 N Ammoniak neutralisiert und aus Methanol umkristallisiert. Man erhält 1.7 g (49 %) der Titelverbindung vom Schmp. >300 ° C.

Beispiel 19

In analoger Weise wie in Beispiel 18 beschrieben erhält man:

| | Bezeichnung | Ausb. % | Schmelzpunkt ° C (Lsg.mittel) |
|---|---|---|---|
| a) | 8,8-Dimethyl-2-(2-methoxy-4-diethylaminophenyl)--5,6,-7,8-tetrahydro-1H-imidazo[4,5-g]chinolin-6-on | 52 | 183-85 $CH_3COOC_2H_5$ |
| b) | 8,8-Dimethyl-2-(3,4-methylendioxyphenyl)-5,6,7,8-tetrahydro-1H-imidazo[4,5-g]chinolin-6-on | 35 | 200-04 $CH_3OH$ |
| c) | 8,8-Dimethyl-2-(4-diethylaminophenyl)-5,6,7,8-tetra--hydro-1H-imidazo[4,5-g]chinolin-6-onxToluolsulfon-säure | 63 | 320 - 21 $CH_3$0H/Essigester |
| d) | 8,8-Dimethyl-2-(4-diethylamino-2-allyl-oxyphenyl)-5-,6,7,8-tetra-hydro-1H-imidazo[4,5-g]chinolin-6-on | 47 | 185 - 86 $CH_3OH$ |

Beispiel 20

8,8-Dimethyl-2-(3-methyl-1H-pyrazol-5-yl)-5,6,7,8-tetrahydro-1H-imidazo[4,5-g]chinolin-6-on

1.5 g (13.6 mmol) 3-Methyl-1H-pyrazol-5-aldehyd wurde mit 4.5 g Natriumhydrogensulfit in 9 ml Wasser 3 Std. bei 60 ° C gerührt. Der eingedampfte Rückstand wurde mit 2.79 g (13.6 mmol) 4,4-Dimethyl-6,7-diamino-1,2,3,4-tetrahydro-chinolin-2-on und 40 ml Ethanol 3 Std. unter Luftdurchleiten bei 60 ° C gerührt. Die Hälfte des Ethanols wurde abdestilliert, Wasser zugesetzt und der abgesaugte Rückstand aus Essigester/$CH_3OH$ umkristallisiert. Man erhält 3.3 g (82.5 %) der Titelverbindung vom Schmp. 242-45 ° C.

Beispiel 21

In analoger Weise wie in Beispiel 20 beschrieben erhält man:

| | Bezeichnung | Ausb. % | Schmelzpunkt ° C (Lsg.mittel) |
|---|---|---|---|
| a) | 8,8-Dimethyl-2-(3-hydroxy-4-methoxyphenyl)-5,6,7,8-tetrahydro-1H-imidazo[4,5-g]chinolin-6-on | 63 | 206 - 11 EtOH/$H_2$0 |
| b) | 8,8-Dimethyl-2-(4-diethylamino-2-hydroxyphenyl)-5,6,7,8-tetrahydro-1H-imidazo[4,5-g] chinolin-6-on | 73 | 280 - 81 $CH_3$0H/$CH_2Cl_2$ |
| c) | 8,8-Dimethyl-2-[4-(1-pyrrolidinyl)phenyl]-5,6,7,8-tetrahydro-1-H-imidazo[4,5-g]chinolin-6-on | 61 | 220 - 21 $CH_3OH$ |
| d) | 8,8-Dimethyl-2-(4-methylmercaptophenyl)-5,6,7,8-tetrahydro--1H-imidazo[4,5-g]chinolin-6-on | 69 | 325 - 30 EtOH |
| e) | 8,8-Dimethyl-2-(4-ethoxyphenyl)-5,6,7,8-tetrahydro-1H-imidazo[4,5-g]chinolin-6-on | 74 | 255 - 59 EtOH |
| f) | 8,8-Dimethyl-2-(4-pentyloxyphenyl)-5,6,7,8-tetrahydro-1H-imidazo[4,5-g]chinolin-6-on | 82 | 251 - 53 Isopropanol |

Beispiel 22

8,8-Dimethyl-2-(4-Hydroxysulfonylphenyl)-5,6,7,8-tetrahydro-1H-imidazo[4,5-g]chinolin-6-on

Analog Bsp. 13 b erhält man aus 1.86 g (9.1 mmol) 4,4-Dimethyl-6,7-diamino-1,2,3,4-tetrahydrochinolin-2-on und 2.5 g (9.2 mmol) 4-(4-Morpholinosulfonyl)benzoesäure in Polyphosphorsäure bei 160 °C 3 g (91 %) der Titelverbindung vom Schmp. >300 °C aus Methanol.

Beispiel 23

2,8,8-Trimethyl-5,6,7,8-tetrahydro-1H-imidazo[4,5-g]chinolin-6-on

3.1 g 6,7-Diamino-4,4-dimethyl-1,2,3,4-tetrahydro-chinolin-2-on und 2.8 ml Acetanhydrid rührte man in 50 ml Ethanol 30 min bei Raumtemperatur. Man gab 20 ml konz. Salzsäure zu und refluxierte 18 h. Man entfernte das Lösungsmittel i. Vak., löste den Rückstand in Wasser, stellte mit Ammoniak alkalisch, saugte ab und wusch mit Wasser. Nach Umkristallisation aus Essigester erhielt man 2.4 g der Titelverbindung mit dem Schmp. 290-294 °C.

Beispiel 24

8,8-Dimethyl-2-phenyl-5,6,7,8-tetrahydro-1H-imidazo[4,5-g]chinolin-6-on

3.0 g 6,7-Diamino-4,4-dimethyl-1,2,3,4-tetrahydrochinolin-2-on und 1.5 ml Benzaldehyd rührte man 20 h in 50 ml Ethanol und 5 ml Eisessig. Man entfernte das Lösungsmittel zur Hälfte i. Vak., versetzte mit 50 ml Wasser und saugte ab. Nach Umkristallisation aus Essigester erhielt man 2.4 g der Titelverbindung mit dem Schmp. 296-298 °C.

Beispiel 25

In analoger Weise wie in Beispiel 24 beschrieben erhält man:

| | Bezeichnung | Ausb. % | Schmelzpunkt °C (Lsg.mittel) |
|---|---|---|---|
| a) | 8,8-Dimethyl-2-(2-thienyl)-5,6,7,8-tetrahydro-1H-imidazo[4,5-g]chinolin-6-on | 51 | >300 EtOH |
| b) | 8,8-Dimethyl-2-(2-allyloxyphenyl)-5,6,7,8-tetrahydro-1H-imidazo[4,5-g]chinolin-6-on | 23 | 233-35 Reinigung an Kieselgel $CH_2Cl_2$:$CH_3OH/NH_3$ = 98:2 |

Beispiel 26

8,8-Dimethyl-2-(4-cyanphenyl)-5,6,7,8-tetrahydro-1H-imidazo[4,5-g]chinolin-6-on

4.1 g 4,4-Dimethyl-6,7-diamino-1,2,3,4-tetrahydrochinolin-2-on und 2.6 g 4-Cyanbenzaldehyd wurden in 100 ml Ethanol 7 Tage unter Lufteinwirkung bei 25°C gerührt. Anschließend wurde auf 10 ml eingedampft, mit 10 ml Wasser versetzt, abgesaugt und aus Ethanol/Wasser (1:1) umkristallisiert. Man erhält 5 g der Titelverbindung vom Schmp. 228-33°C.

Beispiel 27

8,8-Dimethyl-2-(4-aminocarbonylphenyl)-5,6,7,8-tetrahydro-1H-imidazo[4,5-g]chinolin-6-on

3.6 g der in Bsp. 25 erhaltenen Verbindung wurden in 70 ml konzentrierte Schwefelsäure eingetragen und 24 Std. bei 25°C gerührt. Anschließend wurde auf Eis gegossen, mit konzentriertem Ammoniak neutralisiert, abgesaugt und gut mit Wasser gewaschen. Man erhält 1.8 g der Titelverbindung nach Kristallisation aus Ethanol/Wasser vom Schmp. 317-19°C.

Vergleichsbeispiel 28

8,8-Dimethyl-2-(4-pyridyl)-5,6,7,8-tetrahydro-1H-imidazo[4,5-g]chinolin-dihydrochlorid

2.5 g (8.5 mmol) 8,8-Dimethyl-2-(4-pyridyl)-5,6,7,8-tetrahydro-1H-imidazo[4,5-g]chinolin-6-on (Bsp. 5) wurden langsam in eine Suspension von 4.3 g $LiAlH_4$ in 150 ml THF eingetragen und 8 Std. unter Rückfluß erhitzt. Nach Zersetzen von überschüssigem $LiAlH_4$ mit gesättigter NaCl-Lösung wurde 3 x mit Methylenchlorid/Methanol (99:1) extrahiert. Der eingedamfpte Rückstand wurde an Kieselgel (Laufmittel: $CH_2Cl_2$/5 % $CH_3OH$) gereinigt, die gewünschten Fraktionen eingedampft, in Isopropanol gelöst, mit ethanolischer Salzsäure sauer gestellt und die Kristalle abgesaugt. Man erhält 1.0 g der Titelverbindung als Dihydrochlorid vom Schmp. 240-42°C.

Beispiel 29

8,8-Dimethyl-2-(4-diethylamino-2-propyloxyphenyl)-5,6,7,8-tetrahydro-1H-imidazo[4,5-g]chinolin-6-on

2 g 8,8-Dimethyl-2-(4-diethylamino-2-allyloxyphenyl)-5,6,7,8-tetrahydro-1H-imidazo[4,5-g]chinolin-6-on wurden in Ethanol an Palladium/Kohle hydriert. Nach beendeter Wasserstoffaufnahme wurde vom Katalysator abgetrennt, eingeengt und aus Isopropanol umkristallisiert. Man erhält 1,8 g der Titelverbindng vom Schmelzpunkt 147 - 148° C.

Beispiel 30

In analoger Weise wie in Bsp. 26 beschrieben erhält man:

| | Bezeichnung | Ausb. % | Schmelzpunkt °C (Lsg.mittel) |
|---|---|---|---|
| a) | 8,8-Dimethyl-2-phenylmethyl-5,6,7,8-tetrahydro-1H-imidazo-[4,5-g]chinolin-6-on | 65 | 293 - 303 Essigester |
| b) | 8,8-Dimethyl-2-(4-trifluormethylphenyl)-5,6,7,8-tetrahydro-1H-imidazo-[4,5-g]chinolin-6-on | 58 | 325 - 30 nach Säulenchromatographie |
| c) | 8,8-Dimethyl-2-(1-naphthyl)-5,6,7,8-tetrahydro-1H-imidazo-[4,5-g]chinolin-6-on | 68 | 212 - 20 nach Säulenchromatographie |
| d) | 8,8-Dimethyl-2-(4-hydroxyphenyl)-5,6,7,8-tetrahydro-1H-imidazo[4,5-g]chinolin-6-on | 49 | 245 - 49 nach Säulenchromatographie |
| e) | 8,8-Dimethyl-2-(3-hydroxyphenyl)-5,6,7,8-tetrahydro-1H-imidazo[4,5-g]chinolin-6-on | 63 | 335 - 40 Essigester |
| f) | 8,8-Dimethyl-2-(4-tert.-butylphenyl)-5,6,7,8-tetrahydro-1H-imidazo[4,5-g]chinolin-6-on | 72 | 345 - 47 |
| g) | 8,8-Dimethyl-2-(2-furyl)-5,6,7,8-tetrahydro-1H-imidazo[4,5-g]chinolin-6-on | 46 | > 300 EtOH |

| | | | |
|---|---|---|---|
| h) | 8,8-Dimethyl-2-(2-methyl-3-hydroxy-5-hydroxymethylpyridin-4-yl)-5,6,7,8-tetrahydro-1H-imidazo[4,5-g]chinolin-6-on | 48 | > 300 $CH_3OH/H_2O$ |
| i) | 8,8-Dimethyl-2-(4-nitrophenyl)-5,6,7,8-tetrahydro-1H-imidazo[4,5-g]chinolin-6-on | 92 | 352 - 57 EtOH/5 % $H_2O$ |

Beispiel 31

8,8-Dimethyl-2-(4-chlor-2-methoxyphenyl)-5,6,7,8-tetrahydro-1H-imidazo[4,5-g]chinolin-6-on

In analoger Weise wie in Bsp. 2 beschrieben erhält man aus 4-Chlor-2-methoxybenzoylchlorid und 4,4-Dimethyl-6,7-diamino-1,2,3,4-tetrahydrochinolin-2-on (Bsp. 13a) nach Säulenchromatographie 58 % der Titelverbindung vom Schmelzpunkt 300 - 03° C.

Beispiel 32

8,8-Dimethyl-2-(2-indolyl)-5,6,7,8-tetrahydro-1H-imidazo[4,5-g] chinolin-6-on

In analoger Weise wie in Bsp. 2 beschrieben erhält man aus Indol-2-carbonsäurechlorid und 4,4-Dimethyl-6,7-diamino-1,2,3,4-tetrahydrochinolin-2-on (Bsp. 13a) nach Säulenchromatographie 45 % der Titelverbindung vom Schmelzpunkt 264 - 67° C.

Beispiel 33

8,8-Dimethyl-2-(4-methylsulphonyl)-5,6,7,8-tetrahydro-1H-imidazo[4,5-g] chinolin-6-on

3,6 g (11 mmol) 8,8-Dimethyl-2-(4-methylmercaptophenyl)-5,6,7,8-tetrahydro-1H-imidazo[4,5-g]chinolin-6-on (Bsp. 21d) wurden in 40 ml Eisessig mit 7,2 ml $H_2O_2$ 3 Tage bei 25° C gerührt. Anschließend wurde auf 10 ml Volumen eingeengt, mit Ether versetzt, abgesaugt und durch Säulenchromatographie(Laufmittel: $CH_2Cl_2/CH_3OH$/Eisessig, 30: 1 : 0,1) gereinigt. Die eingedampften Fraktionen wurden in Wasser suspendiert, ammoniakalisch gestellt, abgesaugt und mit Wasser gewaschen. Man erhält 2,4 g (59 %) der Titelverbindung vom Schmelzpunkt 219 - 22° C.

Beispiel 34

8,8-Dimethyl-2-(4-methylsulfonyloxyphenyl)-5,6,7,8-tetrahydro-1H-imidazo[4,5-g] chinolin-6-on

2 g (6,5 mmol) 8,8-Dimethyl-2-(4-hydroxyphenyl)-5,6,7,8-tetrahydro-1H-imidazo[4,5-g]chinolin-6-on (Bsp. 30d) wurden in 10 ml Wasser suspendiert, unter Eiskühlung 5 ml (65 mmol) Methansulfonsäurechlorid und 50 ml 2N NaOH im Wechsel so Zugetropft, daß immer ein pH-Wert von etwa 10 - 13 vorliegt. Nach beendeter Reaktion wird abgesaugt, mit Wasser gewaschen und durch Säulenchromatographie(Laufmittel: $CH_2Cl_2$, $CH_3OH$, Essigsäure; 10 : 1 : 0,2) gereinigt. Die eingedampften Fraktionen wurden in Wasser suspendiert, ammoniakalisch gestellt, abgesaugt und mit Wasser gewaschen. Man erhält 1,5 g (68 %) der Titelverbindung vom Schmelzpunkt 209 - 15° C.

Beispiel 35

8,8-Dimethyl-2-(4-propylsulfonyloxyphenyl)-5,6,7,8-tetrahydro-1H-imidazo[4,5-g]chinolin-6-on

In analoger Weise wie in Bsp. 34 beschrieben, erhält man aus 2 g (6,5 mmol) 8,8-Dimethyl-2-(4-hydroxyphenyl)-5,6,7,8-tetrahydro-1H-imidazo[4,5-g]chinolin-6-on und 7,3 ml (65 mmol) 1-Propansulfonsäurechlorid 1,5 g (56 %) der Titelverbindung vom Schmelzpunkt 148 - 56° C.

Beispiel 36

8,8-Dimethyl-2-(4-carboxyphenyl)-5,6,7,8-tetrahydro-1H-imidazo[4,5-g]chinolin-6-on

6,8 g (21,5 mmol) 8,8-Dimethyl-2-(4-cyanophenyl)-5,6,7,8-tetrahydro-1H-imidazo[4,5-g]chinolin-6-on (Bsp. 26) wurden mit 14 g KOH in 100 ml Ethanol 2 Tage zum Rückfluß erhitzt, anschließend im Vakuum eingedampft, in Wasser aufgenommen und mit konzentrierter Salzsäure angesäuert. Man erhält 7,4 g der Titelverbindung als gelbes, amorphes Pulver.

Beispiel 37

8,8-Dimethyl-2-(4-hydroxymethylphenyl)-5,6,7,8-tetrahydro-1H-imidazo[4,5-g]chinolin-6-on

3,35 g (10 mmol) 8,8-Dimethyl-2-(4-carboxyphenyl)-5,6,7,8-tetrahydro-1H-imidazo[4,5-g]chinolin-6-on (Bsp. 36) wurden in 100 ml DMF gelöst und zum Entfernen des anhaftenden Wassers auf 50 ml eingeengt. Nach Zutropfen von 1,4 ml Triethylamin wurden bei 0 - 5 ° C 0,95 ml Chlorameisensäureethylester zugetropft. In diese Lösung tropft man 1 g $NaBH_4$ gelöst in 30 ml DMF bei 5 - 10° C ein. Nach Abdestillieren des DMF wurde mit Wasser versetzt, mit 2n HCl angesäuert, abgesaugt und durch Säulenchromatographie (Laufmittel: $CH_2Cl_2$/mit $NH_3$ gesättigtes $CH_3OH$; 10 : 1) gereinigt. Nach Kristallisation aus Ethanol erhält man 0,8 g (25 %) der Titelverbindung vom Schmelzpunkt 214 - 32° C.

Beispiel 38

8,8-Dimethyl-2-(4-aminophenyl)-5,6,7,8-tetrahydro-1H-imidazo [4,5-g]chinolin-6-on

13 g 8,8-Dimethyl-2-(4-nitrophenyl)-5,6,7,8-tetrahydro-1H-imidazo[4,5-g]chinolin-6-on (Bsp. 30i) wurden an Palladium/Kohle in Methanol hydriert. Nach Abtrennen des Katalysators wurde eingedampft und aus 2N HCl/Ethanol 1 : 1 umkristallisiert. Man erhält 7,8 g der Titelverbindung als Hydrochlorid vom Schmelzpunkt 305 - 08° C Zers.

Beispiel 39

8,8-Dimethyl-2-(4-(1-pyrrolyl)phenyl)-5,6,7,8-tetrahydro-1H-imidazo[4,5-g]chinolin-6-on

3,06 g 8,8-Dimethyl-2-(4-aminophenyl)-5,6,7,8-tetrahydro-1H-imidazo[4,5-g]chinolin-6-on (Bsp. 38) wurden mit 30 ml Eisessig, 5 ml Wasser, 2 Tropfen konzentrierter Salzsäure und 1,32 ml 2,5-Dimethoxytetrahydrofuran 20 Stunden bei 25° C stehen gelassen. Dann mit 100 ml Wasser verdünnt, ammoniakalisch gestellt und mit Wasser gewaschen. Nach Kristallisation aus Ethanol erhält man 0,8 g der Titelverbindung vom Schmelzpunkt 329 - 50° C Zers.

Beispiel 40

8,8-Dimethyl-2-( 4-methoxyphenylamino )-5,6,7,8-tetrahydro-1-H-imidazo[4,5-g]chinolin-6-on

a) 2,7 g (10 mmol) 1,1-Dichlor-1,1-diphenoxymethan wurden in 30 ml Methylenchlorid mit 2,8 ml (20 mmol) Triethylamin vorgelegt und unter Eiskühlung 1,2 g (10 mmol) p-Anisidin, in 10 ml Methylenchlorid gelöst, zugetropft. Nach einer Stunde bei 25° C wurde mit Wasser versetzt, die organische Phase 2 x mit Wasser gewaschen, getrocknet und eingeengt. Man erhält 2,9 g (91 %) N-(4-Methoxyphenyl)-diphenoxyimidocarbonat.

b) 2,9 g (10 mmol) N-(4-Methoxyphenyl)diphenoxyimidocarbonat und 2,0 g (10 mmol) 4,4,-Dimethyl-6,7-diamino-1,2,3,4-tetrahydro-chinolin-2-on (Bsp. 13a) wurden in 100 ml Isopropanol 10 Stunden bei 70° C gerührt. Nach Abdampfen des Lösungsmittels wurde der Rückstand durch Säulenchromatographie (Laufmittel: $CH_2Cl_2/CH_3OH$, 50 : 1 bis 20 : 1) gereinigt. Man erhält 2,4 g (72 %) der Titelverbindung vom Schmelzpunkt: 283 - 87° C.

## Patentansprüche

**1.** Tricyclische Benzimidazole der Formel I

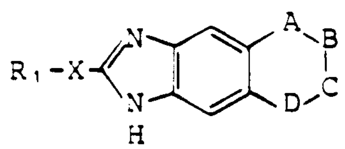

(I),

in welcher

$R_1$ einen Phenylring der allgemeinen Formel II darstellt,

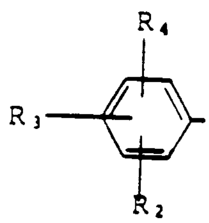

(II),

wobei $R_2$, $R_3$, $R_4$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-Alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe durch ein Schwefel- oder Sauerstoffatom ersetzt sein kann, eine Alkylcarbonylamino, Aminocarbonylamino-oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Hydroxyalkyl-, Dialkylamino-, 1-Imidazolyl-, Trifluormethyl-, Cyan-, Hydroxysulfonyl-, Bishydroxyalkylamino-, 1-Pyrrolidino-, 1-Piperidino-, 4-Morpholino-, 4-Thiomorpholino-, 1-Pyrazolyl-, 1-Triazolyl-, 2-Oxopyrrolidin-yl-, 2-Oxopiperidin- oder Pyrrolylgruppe sein können, und die zuvor genannten Alkylteile der bei $R_2$, $R_3$ und $R_4$ genannten Substituenten 1 - 5 Kohlenstoffatome enthalten,

oder $R_1$ einen Naphthyl- oder Methylendioxyphenylrest,

oder einen gesättigten oder ungesättigten heterocyclischen Fünfring mit 1 - 4 Heteroatomen oder einen heterocyclischen Sechsring mit 1 - 5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere $C_1$-$C_6$-Alkyl-, $C_1$-$C_6$-Alkoxy-, $C_1$-$C_6$-Alkylmercapto-, Hydroxy-, Oxo-, Nitro-, Amino-, Halogen- oder Cyangruppen substituiert oder mit einem Phenylring zu einem Bicyclus kondensiert sein können,

oder für den Fall, daß X eine Bindung darstellt, $R_1$ neben den oben genannten Gruppen auch ein Wasserstoffatom, eine $C_1$-$C_{10}$-Alkyl-, $C_3$-$C_7$-Cycloalkyl-, $C_2$-

$C_{10}$-Alkenyl-, $C_3$-$C_7$-Cycloalkenyl-, $C_2$-$C_{10}$-Alkinyl-, Halogenalkyl-, Alkoxyalkyl-, Carboxyalkyl-, Alkoxycarbonylalkyl-, Aminoalkyl-, Hydroxyalkyl-, eine Hydroxy-, Mercapto-, Amino-, Alkylmercapto-, Alkylcarbonylamino-, Formylamino-, Alkylsufonylamino-, Formylaminoalkyl-, Alkoxycarbonylaminoalkyl-, Alkylsulfonylaminoalkyl-, Trifluormethyl- oder eine Alkylcarbonylaminoalkylgruppe bedeutet, wobei die zuvor unter $R_1$ genannten Alkyl- oder Alkoxygruppen 1-6 Kohlenstoffatome enthalten,

X eine Bindung, eine $C_1$-$C_4$-Alkylen-, die Vinylen-, die Iminogruppe -NH- oder die Amidgruppe -CONH- bedeutet, und

A, B, C und D unabhängig voneinander Kohlenstoff- oder Stickstoffatome bedeuten, wobei ein Atom von A, B, C oder D ein Stickstoffatom bedeutet und die anderen drei Atome Kohlenstoffatome darstellen, und A, B, C oder D durch Wasserstoff, $C_1$-$C_6$-Alkyl- oder $C_3$-$C_7$-Cycloalkylgruppen substituiert sein können, wobei ein Kohlenstoffatom durch eine Hydroxy- oder Oxogruppen substituiert ist oder Bestandteil eines 3- bis 7-gliedrigen Spirocyclus sein kann, und der A, B, C und D enthaltende Sechsring gesättigt, teilweise ungesättigt und ungesättigt sein kann,

deren Tautomere und deren physiologisch verträgliche Salze anorganischer oder organischer Säuren.

**2.** Verbindungen gemäß Anspruch 1, dadurch gekennzeichnet, daß

$R_1$ den Phenylrest der allgemeinen Formel II bedeutet, in der

$R_2$ ein Wasserstoff, die Methansulfonyloxy-, Trifluormethansulfonyloxy-, Methansulfonylamino-, Trifluormethansulfonylamino-, Methansulfonylmethylamino-, Trifluormethansulfonylmethylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-, Trifluormethyl- oder die 1-Imidazolylgruppe,

$R_3$ Wasserstoff, die Methyl-, Methoxy-, Dimethylamino-, Diethylamino- oder Chlorgruppe bedeutet,

$R_4$ Wasserstoff oder die Methoxygruppe ist

oder

$R_1$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxypyridin-, Pyrazin-, N,N'-Dioxypyrazin-, Pyrimidin-, N,N'-Dioxypyrimidin-, Pyridazin-, Oxazin-, Thiazin-, Triazin-, Tetrazin-, Piperidin-, Piperazin-, Morpholin- oder Thiomorpholinrest darstellt, sowie deren Methyl-, Ethyl-, Methoxy-, Ethoxy-, Methylmercapto, Ethylmercapto- und chlorsubstituierten Derivate, oder den Naphthalin, Methylendioxyphenyl-, Indol-, Indazol-, Chinolin- oder Isochinolinrest bedeutet,

oder

$R_1$ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Amino-, Acetylamino- oder Formylaminogruppe bedeutet,

X eine Bindung, eine Methylen-, Vinylen-, Imino- oder Amidgruppe bedeutet und

die Kohlenstoffatome von A, B, C oder D durch Wasserstoff oder Methylgruppen substituiert oder Bestandteil eines Spirocyclus mit 5 C-Atomen sein können.

**3.** Verbindungen gemäß Anspruch 1, dadurch gekennzeichnet, daß das tricyclische Ringsystem ein
5,6,7,8-Tetrahydro-1H-imidazo[4,5-g]chinolin-6-on,
2,3,5,6,7,8-Hexahydro-1H-imidazo[4,5-g]chinolin-2,6-dion,
5,6,7,8-Tetrahydro-1H-imidazo[4,5-g]isochinolin-5-on,
5,6-Dihydro-1H-imidazo[4,5-g]isochinolin-5-on,
5,6,7,8-Tetrahydro-1H-imidazo[4,5-g]isochinolin-5,7-dion,
5,6,7,8-Tetrahydro-1H-imidazo[4,5-g]chinolin-6-on oder
ist
sowie deren im A, B, C und D enthaltenden Teilsystem durch Methyl- oder Hydroxygruppen substituierte Derivate.

**4.** Verbindungen gemäß Anspruch 1, dadurch gekennzeichnet, daß

$R_1$ eine Phenylgruppe, die gegebenenfalls durch Methoxy, Ethoxy, Chlor, Methyl, Hydroxy, Dimethylamino, Diethylamino, Imidazol-1-yl, Allyloxy, Cyan und Aminocarbonyl substituiert sein kann, eine Methylendioxyphenylgruppe oder eine Pyridyl-, Chinolinyl-, Pyridazinyl-, Pyrazolyl- oder Thienylgruppe darstellt, sowie deren durch Methyl substituierten Derivate, oder

$R_1$ für den Fall, daß X eine Bindung darstellt, neben den genannten Gruppen auch eine Methyl-, n-Hexyl- und Hydroxygruppe bedeutet.

**5.** Verfahren zur Herstellung von Verbindungen gemäß einem der Ansprüche 1-4, dadurch gekennzeichnet, daß man

a) Verbindungen der allgemeinen Formel III,

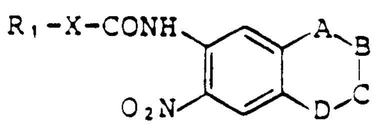

(III),

oder der allgemeinen Formel IV,

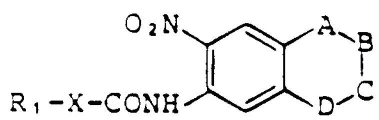

(IV),

in denen $R_1$, X, A, B, C und D die angegebene Bedeutung haben, hydriert und zum Benzimidazol cyclisiert, oder

b) Verbindungen der allgemeinen Formel V,

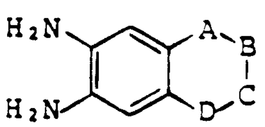

(V),

in denen A, B, C und D die angegebene Bedeutung haben, mit Verbindungen der allgemeinen Formel VI,

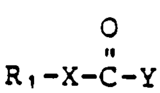

(VI),

in denen $R_1$ und X die angegebene Bedeutung haben und Y ein leicht abspaltbarer Rest ist, umsetzt, oder

c) fuer den Fall, daß $R_1$ in Verbindungen der allgemeinen Formel I eine Amino-, Hydroxy- oder Mercaptogruppe und X ein Valenzstrich bedeutet, Verbindungen der allgemeinen Formel V, in denen A, B, C und D die angegebene Bedeutung haben, mit Reagenzien umsetzt, die Carbonyl-, Thiocarbonyl- oder Iminogruppen uebertragen, wie z.B. Phosgen, Thiophosgen, 1,1'-Carbonyldiimidazol, Chlorameisensaeureester, Harnstoff oder Bromcyan, oder

d) fuer den Fall, daß A und D in Verbindungen der allgemeinen Formel I Stickstoffatome bedeuten, Verbindungen der allgemeinen Formel VII

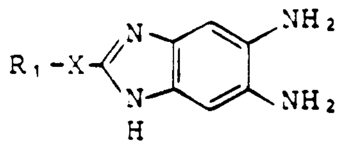

(VII),

in denen $R_1$ und X die angegebenen Bedeutung haben, mit Verbindungen der allgemeinen Formel VIII

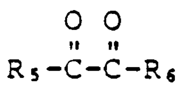

(VIII),

in denen $R_5$ und $R_6$ gleich oder verschieden sein koennen, und Wasserstoff, Alkyl-, Alkoxy-, Halogen- und Hydroxyreste bedeuten, zu Verbindungen der allgemeinen Formel I, in denen B und C Kohlenstoffatome sind, cyclisiert,

anschließend gewuenschtenfalls erhaltene Verbindungen der Formel I in andere Verbindungen der Formel I ueberfuehrt, sowie gewuenschtenfalls die Verbindungen mit anorganischen oder organischen Saeuren in physiologisch vertraegliche Salze ueberfuehrt.

**6.** Arzneimittel enthaltend eine Verbindung der Formel I gemäß den Ansprüchen 1-4 neben üblichen Träger- oder Hilfsstoffen.

**7.** Verwendung von Verbindungen der Formel I gemäß einem der Ansprüche 1-4 zur Herstellung von Arzneimitteln zur Behandlung von Durchblutungsstörungen.

**8.** Verfahren zur Herstellung von Arzneimitteln enthaltend eine Verbindung der Formel I gemäß einem der Ansprüche 1-4, dadurch gekennzeichnet, daß man eine Verbindung der Formel I mit pharmazeutisch üblichen Träger- oder Hilfsstoffen vermischt und zu pharmazeutischen Darreichungsformen verarbeitet.

**Claims**

**1.** Tricyclic benzimidazoles of the formula I

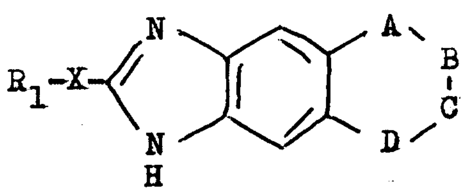

(I)

in which $R_1$ represents a phenyl ring of the general formula II

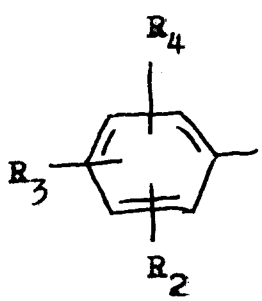

(II)

whereby $R_2$, $R_3$, $R_4$ can be the same or different and in each case can be hydrogen, an alkanesulphonyloxy, trifluoromethylsulphonyloxy, alkanesulphonylamino, trifluoromethanesulphonylamino, N-alkyl-alkanesulphonylamino, N-alkyltrifluoromethanesulphonylamino, alkylsulphenylmethyl, alkylsulphinylmethyl or alkylsulphonylmethyl group, a carbonyl group substituted by a hydroxyl, alkoxy. amino, alkylamino or dialkylamino group, a sulphonyl group substituted by an amino, alkylamino, dialkylamino or cyclic imino group, whereby a methylene group can be replaced by a sulphur or oxygen atom, an alkylcarbonylamino, aminocarbonylamino or alkylaminocarbonylamino group, an alkylmercapto, alkylsulphinyl or alkylsulphonyl group, a nitro, halogen, amino, hydroxyl, alkyl, alkoxy, alkenyloxy, alkynyloxy, cyanoalkyloxy, carboxyalkyloxy, alkoxycarbonylalkyloxy, hydroxyalkyl, dialkylamino, 1-imidazolyl, trifluoromethyl, cyano, hydroxysulphonyl, bis-hydroxyalkylamino, 1-pyrrolidino, 1-piperidino, 4-morpholino, 4-thiomorpholino, 1-pyrazolyl, 1-triazolyl, 2-oxopyrrolidinyl, 2-oxopiperidine or pyrrolyl group and the previously mentioned alkyl parts of the substituents mentioned in the case of $R_2$, $R_3$ and $R_4$ contain 1 - 5 carbon atoms, or $R_1$ represents a naphthyl or methylenedioxyphenyl radical or a saturated or unsaturated heterocyclic five-membered ring with 1 - 4 heteroatoms or a heterocyclic six-membered ring with 1 - 5 heteroatoms, whereby the heteroatoms can be the same or different and signify oxygen, sulphur or nitrogen and, if desired, can carry an oxygen atom on one or more nitrogen atoms and the five- or six-membered rings can possibly be substituted by one or more $C_1$-$C_6$-alkyl, $C_1$-$C_6$-alkoxy, $C_1$-$C_6$-alkylmercapto, hydroxyl, oxo, nitro, amino, halogen or cyano groups or can be condensed with a phenyl ring to give a bicycle or, for the case that X represents a bond, besides the above-mentioned groups, $R_1$ can also signify a hydrogen atom, a $C_1$-$C_{10}$-alkyl, $C_3$-$C_7$-cycloalkyl, $C_2C_{10}$-alkenyl, $C_3$-$C_7$-cycloalkenyl, $C_2$-$C_{10}$-alkynyl, haloalkyl, alkoxyalkyl, carboxyalkyl, alkoxycarbonylalkyl, aminoalkyl, hydroxyalkyl, a hydroxyl, mercapto, amino, alkylmercapto, alkylcarbonylamino, formylamino, alkylsulphonylamino, formylaminoalkyl, alkoxycarbonylaminoalkyl, alkylsulphonylaminoalkyl, trifluoromethyl or an alkylcarbonylaminoalkyl group, whereby the alkyl or alkoxy groups previously mentioned under $R_1$ contain 1 - 6 carbon atoms, X signifies a bond, a $C_1$-$C_4$-alkylene, the vinylene, the imino group -NH- or the amide group -CONH- and A, B, C and D, independently of one another, signify carbon or nitrogen atoms, whereby one atom of A, B, C or D signifies a nitrogen atom and the other three atoms represent carbon atoms, and A, B, C or D can be substituted by hydrogen, $C_1$-$C_6$-alkyl or $C_3$-$C_7$-cycloalkyl groups, whereby one carbon atom is substituted by a hydroxyl or oxo group or can be component of a 3- to 7-membered spirocycle, and the A-, B-, C- and D-containing six-membered ring can be saturated, partly unsaturated and saturated, their tautomers and their physiologically acceptable salts of inorganic or organic acids.

**2.** Compounds according to claim 1, characterised in that $R_1$ signifies a phenyl radical of the general formula II, in which $R_2$ signifies hydrogen, the methanesulphonyloxy, trifluoromethanesulphonyloxy, methanesulphonylamino, trifluoromethanesulphonylamino, methanesulphonylmethylamino, trifluoromethanesulphonylmethylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphinyl, methylsulphonyl, hydroxyl, methyl, methoxy, propargyloxy,cyanomethyloxy, methoxycarbonylmethyloxy, cyano, chloro, nitro, amino, dimethylamino, trifluoromethyl or the 1-imidazolyl group, $R_3$ hydrogen, the methyl, methoxy, dimethylamino, diethylamino or chlorine group, $R_4$ is hydrogen, the methoxy group or $R_1$ signifies the pyrrole, furan, thiophene, pyrazole, imidazole, isothiazole, thiazole, oxazole, triazole, tetrazole, thiadiazole, isoxazole, oxadiazole, pyridine, N-oxypyridine, pyrazine, N,N'-dioxypyrazine, pyrimidine, N,N'-dioxypyrimidine, pyridazine, oxazine, thiazine, triazine, tetrazine, piperidine, piperazine, morpholine or thiomorpholine radical, as well as their methyl-, ethyl-, methoxy-, ethoxy-, methylmercapto-, ethylmercapto-, or chlorine-substituted derivatives or the naphthylene, methylenedioxyphenyl, indole, indazole quinoline or isoquinoline radical or, for the case that X represents a bond, besides the mentioned groups, $R_1$ also signifies a hydrogen atom, the methyl, ethyl, propyl, butyl, pentyl, hexyl, propenyl, cyclopentenyl, cyclohexyl, trifluoromethyl, hydroxyl, mercapto, methylmercapto, amino, acetylamino or formylamino group, X signifies a bond, a methylene, vinylene, imino or amide group and carbon atoms of A, B, C or D can be substituted by hydrogen or methyl groups or can be component of a spirocycle with 5 C-atoms.

**3.** Compounds according to claim 1, characterised in that the tricyclic ring system is a 5,6,7,8-tetrahydro-1H-imidazo[4,5-g]quinolin-6-one, 2,3,5,6,7,8-hexahydro-1H-imidazo[4,5-g]quinoline-2,6-dione, 5,6,7,8-tetrahydro-1H-imidazo[4,5-g]isoquinolin-5-one, 5,6-dihydro-1H- imidazo[4,5-g]isoquinolin-5-one, 5,6,7,8-tetrahydro-1H-imidazo[4,5-g]-isoquinoline-5,7-dione, 5,6,7,8,-tetrahydro-1H-imidazo[4,5-g]-quinolin-6-one, as well as their derivatives substituted by methyl or hydroxyl groups in the A-, B-, C- and D-containing partial system.

**4.** Compounds according to claim 1, characterised in that $R_1$ represents a phenyl group which can possibly be substituted by methoxy, ethoxy, chlorine, methyl, hydroxyl, dimethylamino, diethylamino, imidazol-1-yl, allyloxy, cyano and aminocarbonyl, a methylenedioxyphenyl group or a pyridyl, quinolinyl, pyridazinyl, pyrazolyl or thienyl group, as well as their derivatives substituted by methyl or, for the case that X represents a bond, besides the mentioned groups, $R_1$, also signifies a methyl, n-hexyl and hydroxyl group.

**5.** Process for the preparation of compounds according to one of claims 1-4, characterised in that one
a) hydrogenates compounds of the general formula III

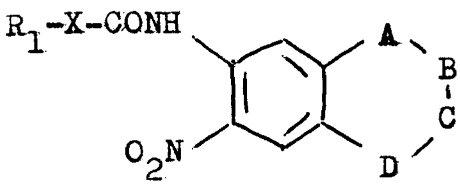

(III)

or of the general formula IV

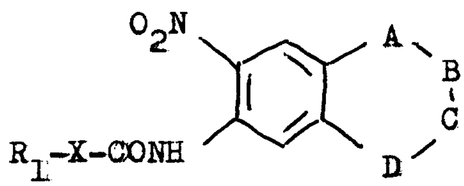

(IV)

in which $R_1$, X, A, D, C and D have the given meanings, and cyclises to the benzimidazole, or
b) reacts compounds of the general formula V

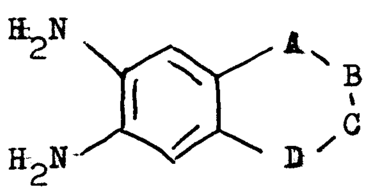

(V)

in which A, B, C and D have the given meanings, with compounds of the general formula VI

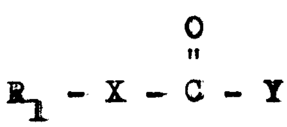

(VI)

in which $R_1$ and X have the given meaning and Y is a residue which is easily split off, or
c) for the case that $R_1$ in compounds of the general formula I signify an amino, hydroxyl or mercapto group and X a valency bond, reacts compounds of the general formula V, in which A, B, C and D have the given meaning, with reagents which transfer the carbonyl, thiocarbonyl or imino group, such as e.g. phosgene, thiophosgene, 1,1'-carbonyldiimidazole, chloroformic acid eaters, urea or cyanogen bromide, or
d) for the case that A and D in compounds of the general formula I signify nitrogen atoms, cyclises compounds of the general formula VII

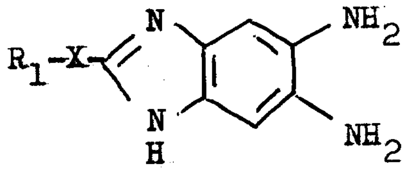

(VII),

in which $R_1$ and X have the given meanings, with compounds of the general formula VIII

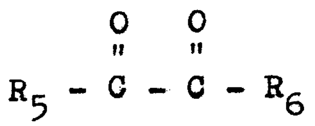

(VIII)

in which $R_5$ and $R_6$ can be the same or different and signify hydrogen, alkyl, alkoxy, halogen and hydroxyl radicals, to give compounds of the general formula I, in which B and C are carbon atoms, subsequently, if desired, converts compounds obtained of the formula I into other compounds of the formula I, as well as, if desired, converts the compounds with inorganic or organic acids into physiologically compatible salts.

6. Medicaments containing a compound of the formula I according to claims 1 - 4, besides usual carrier or adjuvant materials.

7. Use of compounds of the formula I according to one of claims 1 - 4 for the preparation of medicaments for the treatment of circulatory disturbances.

8. Process for the preparation of medicaments containing a compound of the formula I according to one of claims 1 - 4, characterised in that one mixes a compound of the formula I with pharmaceutically usual carrier or adjuvant materials and works up to pharmaceutical forms of administration.

**Revendications**

1. Benzimidazoles tricycliques de formule I

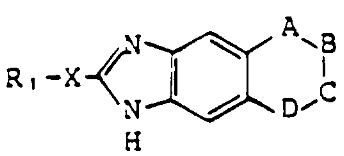

(I)

dans laquelle

$R_1$ représente un noyau phényle de formule générale II

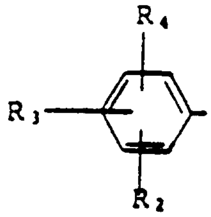

(II)

dans laquelle

$R_2$ $R_3$, $R_4$ peuvent être identiques ou différents et représentent chacun un atome d'hydrogène, un groupe alcanesulfonyloxy, trifluorométhanesulfonyloxy, alcanesulfonylamino, trifluorométhanesulfonylamino, N-alkyl-alcanesulfonylamino, N-alkyl-trifluorométhanesulfonylamino, alkylsulfénylméthyle, alkylsulfinylméthyle ou alkylsulfonylméthyle, un groupe carbonyle substitué par un groupe hydroxy, alcoxy, amino, alkylamino ou dialkylamino, un groupe sulfonyle substitué par un groupe amino, alkylamino, dialkylamino ou iminocyclique, un groupe méthylène pouvant être substitué par un atome de soufre ou un atome d'oxygène, un groupe alkylcarbonylamino, aminocarbonylamino ou alkylaminocarbonylamino, un groupe alkylmercapto, alkylsulfinyle ou alkylsulfonyle, un groupe nitro, halogéno, amino, hydroxy, alkyle, alcoxy, alcényloxy, alcynyloxy, cyanoalkyloxy, carboxyalkyloxy, alcoxycarbonylalkyloxy, hydroxyalkyle, dialkylamino, 1-imidazolyle, trifluorométhyle, cyano, hydroxysulfonyle, bishydroxyalkylamino, 1-pyrrolidino, 1-pipéridino, 4-morpholino, 4-thiomorpholino, 1-pyrazolyle, 1-triazolyle, 2-oxopyrrolidinyle, 2-oxopipéridine ou pyrrolyle, et les parties alkyle mentionnées précédemment des substituants mentionnés pour $R_2$, $R_3$ et $R_4$ comportent 1 - 5 atomes de carbone,

ou $R_1$ représente un groupe naphtyle ou méthylènedioxyphényle,

ou un hétérocycle à cinq chaînons, saturé ou insaturé, comportant 1 - 4 hétéroatomes, ou un hétérocycle à six chaînons comportant 1 - 5 hétéroatomes, les hétéroatomes pouvant être identiques ou différents et représentent un atome d'oxygène, de soufre ou d'azote, et éventuellement, un ou plusieurs atomes d'azote peuvent porter un atome d'oxygène, et les cycles à cinq ou six chaînons peuvent être substitués éventuellement par un ou plusieurs groupes alkyle($C_1$-$C_6$),alcoxy($C_1$-$C_6$), alkyl($C_1$-$C_6$)-mercapto, hydroxy, oxo, nitro, amino, halogéno ou cyano, ou peuvent être condensés avec un noyau phényle en un bicycle,

ou dans le cas où X représente une liaison, $R_1$ peut représenter, en plus des groupes mentionnés ci-dessus, également un atome d'hydrogène, un groupe alkyle($C_1$-$C_{10}$), cycloalkyle($C_3$-$C_7$), alcényle($C_2$-$C_{10}$), cycloalcényle($C_3$-$C_7$), alcynyle($C_2$-$C_{10}$), halogénoalkyle, alcoxyalkyle, carboxyalkyle, alcoxycarbonylalkyle, aminoalkyle, hydroxyalkyle, un groupe hydroxy, mercapto, amino, alkylmercapto, alkylcarbonylamino, formylamino, alkylsulfonylamino, formylaminoalkyle, alcoxycarbonylaminoalkyle, alkylsulfonylaminoalkyle, trifluorométhyle ou un groupe alkylcarbonylaminoalkyle, les groupes alkyle ou alcoxy mentionnés précédemment pour $R_1$ comportant 1 - 6 atomes de carbone,

X représente une liaison, un groupe alkylène($C_1$-$C_4$), vinylène, imino -NH- ou amido -CONH-, et

A, B, C et D représentent indépendamment un atome de carbone ou un atome d'azote, un des atomes parmi A, B, C ou D représentant un atome d'azote et les trois autres atomes représentant un atome de carbone, et A, B, C ou D pouvant être substitués par un atome d'hydrogène, un groupe alkyle($C_1$-$C_6$) ou cycloalkyle($C_3$-$C_7$), un atome de carbone étant substitué par un groupe hydroxy ou oxo, ou pouvant entrer dans la constitution d'un spirocycle à 3 à 7 chaînons, et le cycle à six chaînons contenant A, B, C et D pouvant être saturé, partiellement insaturé ou insaturé,

leurs tautomères et leurs sels physiologiquement acceptables formés avec des acides organiques ou inorganiques.

**2.** Composés selon la revendication I, caractérisés en ce que

$R_1$ représente un groupe phényle de formule générale II, dans laquelle

$R_2$ représente un atome d'hydrogène, un groupe méthanesulfonyloxy, trifluorométhanesulfonyloxy, méthanesulfonylamino, trifluorométhanesulfonylamino, méthanesulfonylméthylamino, trifluorométhanesulfonylméthylamino, méthylsulfénylméthyle, méthylsulfinylméthyle, méthylsulfonylméthyle, aminocarbonyle, aminosulfonyle, méthylaminosulfonyle, diméthylaminosulfonyle, acétylamino, méthylmercapto, méthylsulfinyle, méthylsulfonyle, hydroxy, méthyle, méthoxy, propargyloxy, cyanométhyloxy, méthoxycarbonylméthyloxy, cyano, chloro, nitro, amino, diméthylamino, trifluorométhyle ou 1-imidazolyle,

$R_3$ représente un atome d'hydrogène, un groupe méthyle, méthoxy, diméthylamino, diéthylamino ou chloro,

$R_4$ représente un atome d'hydrogène ou un groupe méthoxy,

ou

$R_1$ représente un groupe pyrrole, furane, thiophène, pyrazole, imidazole, isothiazole, thiazole, oxazole, triazole, tétrazole, thiadiazole, isoxazole, oxadiazole, pyridine, N-oxypyridine, pyrazine, N,N'-dioxy-pyrazine, pyrimidine, N,N'-dioxypyrimidine, pyridazine, oxazine, thiazine, triazine, tétrazine, pipéridine, pipérazine, morpholino ou thiomorpholino, ainsi que leurs dérivés à substituant méthyle, éthyle, méthoxy, éthoxy, méthylmercapto, éthylmercapto et chloro, ou le groupe naphtalène, méthylènedioxyphényle, indole, indazole, quinoléine ou isoquinoléine,

ou

$R_1$ représente, dans le cas où X représente une liaison, en plus des groupes mentionnés, également un atome d'hydrogène, un groupe méthyle, éthyle, propyle, butyle, pentyle, hexyle, propényle, cyclopentényle, cyclohexyle, trifluorométhyle, hydroxy, mercapto, méthylmercapto, amino, acétylamino ou formylamino,

X représente une liaison, un groupe méthylène, vinylène, imino ou amido, et

les atomes de carbone parmi A, B, C ou D peuvent être substitués par un atome d'hydrogène ou un groupe méthyle ou peuvent être des constituants d'un spirocycle comportant 5 atomes de C.

**3.** Composés selon la revendication 1, caractérisés en ce que le système tricyclique est un des composés
5,6,7,8-tétrahydro-1H-imidazo[4,5-g]-quinoléine-6-one,
2,3,5,6,7,8-hexahydro-1H-imidazo[4,5-g]-quinoléine-2,6-dione,
5,6,7,8-tétrahydro-1H-imidazo[4,5-g]-isoquinoléin-5-one,
5,6-dihydro-1H-imidazo[4,5-g]-isoquinoléin-5-one,
5,6,7,8-tétrahydro-1H-imidazo[4,5-g]-isoquinoléin-5,7-dione,
5,6,7,8-tétrahydro-1H-imidazo[4,5-g]- quinoléin-6-one
ainsi que leurs dérivés à substituant méthyle ou hydroxy, dans le système partiel contenant A, B, C et D.

**4.** Composés selon la revendication 1, caractérisés en ce que

$R_1$ représente un groupe phényle qui peut être éventuellement substitué par un groupe méthoxy, éthoxy, chloro, méthyle, hydroxy, diméthylamino, diéthylamino, imidazol-1-yle, allyloxy, cyano et aminocarbonyle, un groupe méthylène-dioxyphényle ou un groupe pyridyle, quinoléinyle, pyridazinyle, pyrazolyle ou thiényle, ainsi que leurs dérivés à substituant méthyle, ou

$R_1$ représente, dans le cas où X représente une liaison, en plus des groupes mentionnés ci-dessus, également un groupe méthyle, n-hexyle ou hydroxy.

**5.** Procédé de préparation des composés selon l'une quelconque des revendications 1-4, caractérisé en ce que

a) on hydrogène des composés de formule générale III,

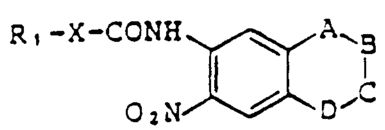

(III)

ou de formule générale IV,

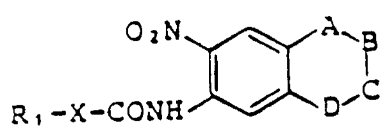

(IV)

dans lesquelles $R_1$, X, A, B, C et D ont les significations mentionnées, on hydrogène et cyclise en benzimidazole, ou

b) on fait réagir des composés de formule générale V

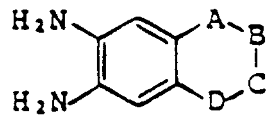

(V)

dans laquelle A, B, C et D ont les significations mentionnées, avec des composés de formule générale VI,

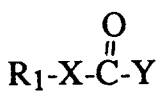

(VI)

dans laquelle $R_1$ et X ont les significations mentionnées et Y représente un reste se séparant facilement, ou

c) dans le cas où $R_1$, dans les composés de formule générale I, représente un groupe amino, hydroxy ou mercapto et X représente une liaison, on fait réagir des composés de formule générale V, dans laquelle A, B, C et D ont la signification mentionnée, avec des partenaires réactionnels capables de transférer un groupe carbonyle, thiocarbonyle ou imino, tels que par exemple le phosgène, le thiophosgène, le 1,1'-carbonyldiimidazole, les chloroformiates, l'urée ou le bromocyan, ou

d) dans le cas où A et D, dans les composés de formule générale I, représentent un atome d'azote, on cyclise des composés de formule générale VII

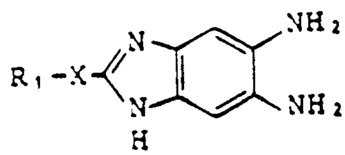

(VII)

dans laquelle $R_1$ et X ont les significations mentionnées, avec des composés de formule générale VIII

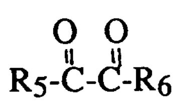

(VIII)

dans laquelle $R_5$ et $R_6$ peuvent être identiques ou différents et représentent un atome d'hydrogène, un groupe alkyle, alcoxy, halogéno ou hydroxy, pour obtenir des composés de formule générale I dans laquelle B et C représentent un atome de carbone,

et ensuite, on transforme éventuellement les composés de formule I obtenus en d'autres composés de formule I, et éventuellement, on transforme ces composés en sels physiologiquement acceptables, avec des acides organiques ou inorganiques.

**6.** Médicament contenant un composé de formule I selon les revendications 1-4, ainsi que des adjuvants et véhicules usuels.

**7.** Utilisation des composés de formule I selon l'une quelconque des revendications 1-4, pour la préparation de médicaments destinés au traitement des troubles de la circulation sanguine.

8. Procédé de préparation de médicaments contenant un composé de formule I selon l'une quelconque des revendications 1-4, caractérisé en ce que l'on mélange un composé de formule I avec des véhicules et adjuvants pharmaceutiques usuels et le transforme en produit galénique.